# EUROPÄISCHE PATENTSCHRIFT

(11) **EP 4 079 211 B1**
(45) Veröffentlichungstag und Bekanntmachung des Hinweises auf die Patenterteilung: **14.10.2026**
(21) Anmeldenummer: 22167047.4
(22) Anmeldetag: 07.04.2022
(51) Int. Cl.: A61B 1/00, A61B 1/05

(54) **VERFAHREN ZUM ERGÄNZEN EINES BAUTEILS EINES MEDIZINISCHEN INSTRUMENTS UND MEDIZINISCHES INSTRUMENT**
METHOD FOR COMPLEMENTING A COMPONENT OF A MEDICAL INSTRUMENT AND MEDICAL INSTRUMENT
PROCÉDÉ PERMETTANT DE COMPLÉTER UN COMPOSANT D'UN INSTRUMENT MÉDICAL ET INSTRUMENT MÉDICAL

(30) Priorität: 19.04.2021 DE 102021109865
(43) Veröffentlichungstag der Anmeldung: 26.10.2022
(73) Patentinhaber: Karl Storz SE & Co. KG, 78532 Tuttlingen (DE)
(72) Erfinder: HEDEMANN, Lars, 78532 Tuttlingen (DE); HENI, Andreas, 78532 Tuttlingen (DE); KUPFERSCHMID, Markus, 78532 Tuttlingen (DE)

(56) Entgegenhaltungen:
- US-A1- 2011 288 372
- US-A1- 2016 316 997
- US-A1- 2019 246 884

## Beschreibung

Die vorliegende Erfindung ist auf ein Verfahren zum Herstellen eines medizinischen Instruments, nämlich zum Ergänzen eines Bauteils eines medizinischen Instruments durch ein Kunststoff-Bauteil bezogen. Die vorliegende Erfindung ist ferner auf ein Gusswerkzeug zum Ergänzen eines Bauteils eines medizinischen Instruments durch ein Kunststoff-Bauteil, bezogen. Die vorliegende Erfindung ist ferner auf ein medizinisches Instrument bezogen, das insbesondere mittels des Verfahrens und/oder mithilfe des Gusswerkzeugs hergestellt sein kann.

Einige medizinische Instrumente weisen einen komplexen Aufbau und eine komplexe Geometrie auf. Das distale Ende eines starren oder teilweise oder vollständig flexiblen Endoskops ist ein Beispiel. Eine Lichtquelle zum Erzeugen von Beleuchtungslicht oder ein Ende eines Lichtwellenleiters zum Übertragen von Beleuchtungslicht zu dem distalen Ende, ein Objektiv, immer häufiger auch ein Bildsensor sowie die distalen Enden von einem oder mehreren Arbeits- und/oder Spülkanälen sind auf engem Raum angeordnet und mechanisch dauerhaft und robust verbunden. In der Regel soll ferner auch am distalen Ende die Oberfläche des Endoskops - von Ein- und Ausgängen von Fluid- und Arbeitskanälen abgesehen - fluiddicht oder hermetisch dicht sein.

Ein Ansatz besteht in einer Verwendung eines Kappen-Bauelements mit Öffnungen, in die Lichtquelle, Kamera etc. eingesetzt werden. Ein anderer technischer Ansatz besteht darin, die Bauteile oder Baugruppen am distalen Ende eines Endoskops zu umspritzen, also in einem Spritzgießverfahren zu umhüllen. Dabei entstehen jedoch Temperaturgradienten und Temperaturen, die elektronische Bauelemente schädigen können.

In US 2011/0288372 A1 sind ein Endoskop und ein Verfahren zum Herstellen eines Endoskops beschrieben, bei dem eine Kameraeinrichtung und eine Lichtquelle in das Material des Gehäuses eingebettet werden. Die Kameraeinrichtung, die Lichtquelle und zugeordnete elektronische Bauteile sind an einer "Tragstruktur/Platine" befestigt.

In US 2019/246884 A1 ist ein Endoskop mit einer außermittigen Blickrichtung beschrieben. In das distale Ende einer länglichen Kanüle ist ein Bauteil mit einem gesonderten Träger, an dem ein Kameramodul und LEDs befestigt sind, vorgesehen.

In US 2016/316997 A1 sind eine Vorrichtung und ein Verfahren zum Herstellen eines länglichen hohlen Profilelements eines Endoskops beschrieben. Das Endoskop weist einen Endoskopkopf, in dem eine Kamera und LEDs angeordnet sind, auf. Proximal schließt sich ein längliches hohles Profilelement an, in dem mehrere Zugseile angeordnet sind. Am proximalen Ende des länglichen hohlen Profilelements ist ein Zwischenteil angeordnet, in dem Spiralhülsen für die Zugseile verankert sind. In dem Biegebereich ist ein notwendigerweise elastisches Schlauchelement vorgesehen. Das distale Ende des Schlauchelements ist in einen im Durchmesser erweiterten Bereich des Arbeitskanals in dem Endoskopkopf eingesetzt. Das Schlauchelement ist in einer axialen zentrischen inneren Öffnung des Zwischenteils, durch die der Arbeitskanal läuft, angeordnet.

Eine Aufgabe der vorliegenden Erfindung besteht darin, eine verbesserte Herstellung eines medizinischen Instruments zu ermöglichen.

Diese Aufgabe wird gelöst durch die Gegenstände der unabhängigen Ansprüche.

Weitere Ausführungsformen sind in den abhängigen Ansprüchen definiert.

Ausführungsformen der vorliegenden Erfindung beruhen auf der Idee, anstatt eines thermoplastischen Kunststoffs einen ursprünglich bei Raumtemperatur flüssigen Kunststoff zu verwenden, dessen Verfestigung oder Aushärtung durch elektromagnetische Strahlung, beispielsweise blaues oder ultraviolettes Licht, bewirkt oder ausgelöst wird. Ferner beruhen Ausführungsformen der vorliegenden Erfindung auf der Idee, eine Baugruppe - beispielsweise aus Lichtquelle und Kamera - bereits vor dem Umgießen mit Kunststoff mechanisch mit dem distalen Ende eines Schaftrohrs zu verbinden.

Ein Verfahren zum Ergänzen eines Bauteils eines medizinischen Instruments durch ein Kunststoff-Bauteil umfasst Schritte des Einlegens des Bauteils des medizinischen Instruments in ein Gusswerkzeug und des Schließens des Gusswerkzeugs, wobei ein erster Bereich einer Formoberfläche des Gusswerkzeugs an das Bauteil angelegt wird und ein Gussraum zwischen einem zweiten Bereich der Formoberfläche des Gusswerkzeugs und dem Bauteil verbleibt; einen Schritt des Zuführens eines flüssigen Kunststoffs zu dem Gussraum; und einen Schritt des Einkoppelns elektromagnetischer Strahlung in den flüssigen Kunststoff innerhalb des Gussraums, um eine Verfestigung des Kunststoffs zu bewirken oder auszulösen und das Kunststoff-Bauteil zu bilden.

Das Verfahren ist insbesondere zum Ergänzen eines Schafts oder Schaftrohrs eines Endoskops oder eines anderen metallischen Bauteils geeignet. Ferner kann das Verfahren zum Ergänzen eines Bauteils aus einem Kunststoff oder einem anderen Material geeignet sein.

Das Gusswerkzeug kann mehrere Werkzeugteile aufweisen, zwischen denen im geschlossenen Zustand ein Hohlraum verbleibt. Dieser Hohlraum wird von einer im Folgenden als Formoberfläche bezeichneten inneren Oberfläche des Gusswerkzeugs begrenzt. Nach dem Einlegen des Bauteils des medizinischen Instruments in das Gusswerkzeug nimmt das Bauteil einen Teil des Hohlraums ein. Dabei liegt der erste Bereich der Formoberfläche flächig oder linienförmig an dem Bauteil an.

Der Gussraum wird außer durch den zweiten Bereich der Formoberfläche auch durch einen nicht an der Formoberfläche des Gusswerkzeugs anliegenden Oberflächenbereich des Bauteils gebildet. Bei dem Zuführen des flüssigen Kunststoffs zu dem Gussraum wird der Gussraum insbesondere vollständig gefüllt. Der Gussraum kann in ein Lumen oder einen Hohlraum innerhalb des Bauteils übergehen. Dieses Lumen oder dieser Hohlraum kann bei dem Verfahren ganz oder nur teilweise gefüllt werden.

Das Einkoppeln elektromagnetischer Strahlung umfasst insbesondere das Einkoppeln ultravioletten Lichts oder sichtbaren Lichts in dem vom gesunden menschlichen Auge als blau wahrgenommenen Spektralbereich. Die elektromagnetische Strahlung kann unmittelbar eine Verfestigung, beispielsweise eine Polymerisation, bewirken oder eine solche nur auslösen, indem beispielsweise ein Katalysator erzeugt oder freigesetzt wird, der eine Polymerisation oder eine andere chemische Reaktion bewirkt.

Insbesondere die Verwendung eines bei Raumtemperatur flüssigen Kunststoffs, der nicht wie ein Thermoplast durch Abkühlen in den festen Zustand übergeht, sondern durch einen chemischen Prozess, der durch elektromagnetische Strahlung ausgelöst oder getrieben wird, kann die thermische Belastung deutlich vermindern oder vermeiden. Deshalb können in das Gusswerkzeug mit dem Bauteil beispielsweise auch elektronische Bauelemente eingesetzt werden, die durch die Temperaturen und Temperaturgradienten, die bei herkömmlichen Gussverfahren mit Thermoplasten entstehen, geschädigt würden.

Bei einem Verfahren, wie es hier beschrieben ist, wird die elektromagnetische Strahlung insbesondere durch einen für die elektromagnetische Strahlung transparenten Formeinsatz in einem Werkzeugteil oder durch ein transparentes Werkzeugteil oder durch einen transparenten Schieber oder durch einen transparenten Einsatz in einem Schieber zum Verschließen einer Zuführöffnung in den flüssigen Kunststoff eingekoppelt.

Ein transparenter Formeinsatz in einem Werkzeugteil bildet einen Teil der Formoberfläche des Gusswerkzeugs, schließt also an den Gussraum unmittelbar an. Eine in das Gusswerkzeug integrierte Lichtquelle oder außerhalb des Gusswerkzeugs angeordnete und mit dem transparenten Formeinsatz optisch gekoppelte Lichtquelle kann die Verfestigung des flüssigen Kunststoffs auslösendes oder bewirkendes Licht erzeugen, das durch den transparenten Formeinsatz hindurch in den flüssigen Kunststoff gelangt.

Für ein transparentes Werkzeugteil, das nicht nur einen transparenten Formeinsatz aufweist, sondern vollständig für die elektromagnetische Strahlung transparent ist, gilt Entsprechendes.

Ein transparenter Schieber oder ein Schieber mit einem transparenten Einsatz ist beispielsweise in einer Bohrung oder einem anderen zylindrischen Kanal, der in den Gussraum übergeht, bewegbar angeordnet. Die Öffnung der Bohrung oder des zylindrischen Kanals zu dem Gussraum bildet die Zuführöffnung zum Zuführen von flüssigem Kunststoff in den Gussraum. Das dem Gussraum zugewandte Ende des Schiebers verschließt die Zuführöffnung - von einem für eine mechanische Leichtgängigkeit erforderlichen geometrischen Spiel abgesehen - vollständig. Ein dem Gussraum zugewandter Teilbereich der äußeren Oberfläche des Schiebers, der an den Gussraum angrenzt, bildet einen Teil der Formfläche des Gusswerkzeugs.

Der transparente Schieber oder der transparente Einsatz des Schiebers kann mit einer Lichtquelle oder einer anderen Quelle zur Erzeugung der elektromagnetischen Strahlung beispielsweise durch einen Lichtwellenleiter oder durch andere optische Elemente optisch gekoppelt sein. Die Lichtquelle oder die andere Quelle elektromagnetischer Strahlung kann mit dem Schieber mechanisch starr gekoppelt oder in diesen integriert sein.

Wenn der Schieber (weitgehend) aus einem transparenten Material gebildet ist, ist die Mantelfläche des Schiebers insbesondere beschichtet, um dort einen Austritt der elektromagnetischen Strahlung in einen den Schieber umgebenden dünnen Spalt und damit eine Verfestigung von dort vorliegendem flüssigen Kunststoff zu verhindern.

Ein transparenter Formeinsatz oder ein transparentes Werkzeugteil kann eine großflächige und gleichmäßige Einkopplung der elektromagnetischen Strahlung in den Gussraum ermöglichen. Die Verwendung eines transparenten Schiebers oder eines Schiebers mit einem transparenten Einsatz kann eine im Übrigen herkömmliche oder weitgehend herkömmliche Ausgestaltung des Gusswerkzeugs erlauben.

Bei einem Verfahren, wie es hier beschrieben ist, wird der flüssige Kunststoff insbesondere von einem von dem Gussraum abgewandten Ende des Bauteils aus durch dieses hindurch dem Gussraum zugeführt.

Insbesondere wenn das Bauteil als Rohr ausgebildet ist oder eine Durchgangsbohrung oder einen anderen Kanal aufweist, die oder der bis zu dem Gussraum reicht und andererseits aus dem Gusswerkzeug herausreicht oder zumindest bis an die äußere Oberfläche des Gusswerkzeugs reicht, kann durch das Lumen des Rohrs oder der Durchgangsbohrung oder des Kanals hindurch flüssiger Kunststoff dem Gussraum zugeführt werden. In diesem Fall muss das Gusswerkzeug keinen Zuführkanal zum Zuführen flüssigen Kunststoffs aufweisen.

Bei einem Verfahren, wie es hier beschrieben ist, wird die elektromagnetische Strahlung insbesondere von einem von dem Gussraum abgewandten Ende des Bauteils her durch dieses hindurch in den Hohlraum eingekoppelt.

Insbesondere wenn das Bauteil als Rohr ausgebildet ist oder eine Durchgangsbohrung oder einen anderen Kanal aufweist, die oder der bis zu dem Gussraum reicht und andererseits aus dem Gusswerkzeug herausreicht, kann durch das Lumen des Rohrs oder der Durchgangsbohrung oder des Kanals hindurch oder zumindest bis an die äußere Oberfläche des Gusswerkzeugs reicht, kann ein Einkoppeln der elektromagnetischen Strahlung in den flüssigen Kunststoff in dem Gussraum durch das Bauteil hindurch möglich sein. Ein transparenter Formeinsatz in einem Werkzeugteil oder ein transparentes Werkzeugteil oder ein transparenter Schieber oder ein transparenter Einsatz in einem Schieber kann in diesem Fall überflüssig sein.

Bei einem Verfahren, wie es hier beschrieben ist, umfasst das Einlegen des Bauteils insbesondere ein Einlegen einer mit dem Bauteil mechanisch verbundenen Baugruppe in das Gusswerkzeug.

Das Bauteil ist insbesondere ein Schaftrohr, beispielsweise ein Schaftrohr eines Endoskops oder eines Exoskops.

Ein Verfahren zum Ergänzen eines Schaftrohrs und einer Baugruppe eines medizinischen Instruments durch ein Kunststoff-Bauteil umfasst einen Schritt des Bereitstellens eines Schaftrohrs mit einem distalen Ende, ein mechanisches Verbinden einer Baugruppe mit dem distalen Ende des Schaftrohrs durch eine Verbindungseinrichtung, Schritte des Einlegens des Schaftrohrs mit der Baugruppe in ein Gusswerkzeug und des Schließens des Gusswerkzeugs, wobei ein vorbestimmter Bereich der äußeren Oberfläche der Baugruppe an einer Formoberfläche des Gusswerkzeugs anliegt, einen Schritt des Zuführens eines flüssigen Kunststoffs in das Gusswerkzeug, wobei der flüssige Kunststoff das distale Ende des Schaftrohrs und die äußere Oberfläche der Baugruppe benetzt, und einen Schritt des Verfestigens des flüssigen Kunststoffs, wobei der verfestigte Kunststoff das Kunststoff-Bauteil bildet und eine weitere mechanische Verbindung der Baugruppe mit dem distalen Ende des Schaftrohrs schafft.

Das medizinische Instrument ist insbesondere ein Endoskop oder ein Exoskop. Das Schaftrohr bildet insbesondere die mechanisch tragende Struktur des Schafts des Endoskops.

Das Schaftrohr ist insbesondere aus Metall gebildet. Alternativ kann das Schaftrohr aus einem Kunststoff oder einem anderen Material gebildet sein. Das Schaftrohr kann gerade oder gekrümmt, vollständig starr oder teilweise oder vollständig flexibel sein. Die Baugruppe kann ein oder mehrere Bauelemente umfassen. Der vorbestimmte Bereich der äußeren Oberfläche der Baugruppe umfasst insbesondere eine Lichtaustrittsfläche einer Lichtquelle oder eine Lichtaustrittsfläche an einem distalen Ende eines Lichtwellenleiters und/oder eine Lichteintrittsfläche eines Objekts oder einer Kamera zum Erzeugen und Erfassen eines Bilds.

Das Gusswerkzeug umfasst insbesondere mehrere Werkzeugteile, die einen Hohlraum umschließen, und deren innere Oberflächen die den Hohlraum begrenzende Formoberfläche bilden. Nach dem Einlegen des Schaftrohrs in das Gusswerkzeug und dem Schließen des Gusswerkzeugs ragt das Schaftrohr insbesondere aus dem Gusswerkzeug heraus oder reicht bis zu einer äußeren Oberfläche des Gusswerkzeugs. Alternativ kann das Schaftrohr vollständig innerhalb des Hohlraums des Gusswerkzeugs angeordnet sein. Nach dem Schließen des Gusswerkzeugs liegt insbesondere ein lateraler Oberflächenbereich des Schaftrohrs an einem ersten Bereich der Formoberfläche des Gusswerkzeugs an. Ein zweiter Bereich der Formoberfläche des Gusswerkzeugs begrenzt zusammen mit einem weiteren, nicht an der Formoberfläche des Gusswerkzeugs anliegenden Bereich der Oberfläche des Schaftrohrs einen Gussraum, dem der flüssige Kunststoff zugeführt und in dem der flüssige Kunststoff verfestigt wird.

Wenn der flüssige Kunststoff ein thermoplastischer Kunststoff ist, wird er durch Abkühlen verfestigt. Alternativ kann der flüssige Kunststoff auf andere Weise verfestigt werden, beispielsweise durch Polymerisation. In diesem Fall kann das Verfestigen durch Einstrahlen von ultraviolettem Licht oder von Licht in dem vom gesunden menschlichen Auge als blau wahrgenommenen Spektralbereich oder von anderer elektromagnetischer Strahlung ausgelöst oder bewirkt werden.

Die mechanische Verbindung der Baugruppe mit dem distalen Ende des Schaftrohrs durch die Verbindungseinrichtung macht eine separate mechanische Fixierung, beispielsweise durch einen Schieber oder durch ein Merkmal der Gussform überflüssig. Nachteile wie nach dem Guss verbleibende Hohlräume oder Ausnehmungen, die bis an die Baugruppe heran reichen, können dadurch vermieden werden.

Es können eine einzige Verbindungseinrichtung oder mehrere Verbindungseinrichtungen zur mechanischen des distalen Endes des Schaftrohrs mit einer oder mehreren Baugruppen vorgesehen werden. Beispielsweise können eine erste Verbindungseinrichtung zur mechanischen Verbindung einer Baugruppe, die mindestens einen Bildsensor umfasst, mit dem distalen Ende des Schaftrohrs und eine zweite Verbindungseinrichtung zur mechanischen Verbindung einer Baugruppe, die mindestens eine Leuchtdiode oder eine andere Lichtquelle umfasst, mit dem distalen Ende des Schaftrohrs vorgesehen sein.

Bei einem Verfahren, wie es hier beschrieben ist, umfasst insbesondere zumindest entweder das Einlegen des Schaftrohrs mit der Baugruppe oder das Schließen des Gusswerkzeugs ein elastisches Verformen der Verbindungseinrichtung, sodass eine elastische Rückstellkraft der Verbindungseinrichtung den vorbestimmten Bereich der äußeren Oberfläche der Baugruppe gegen die Formoberfläche des Gusswerkzeugs presst.

Die elastische Verformung kann gering sein. Die elastische Verformung ist insbesondere lediglich so dimensioniert, dass selbst innerhalb der Fertigungstoleranzen des Schaftrohres, der Verbindungseinrichtung und der Baugruppe der vorbestimmte Bereich der äußeren Oberfläche der Baugruppe zuverlässig an der Formoberfläche des Gusswerkzeugs anliegt.

Durch das Anliegen des vorbestimmten Bereichs der äußeren Oberfläche der Baugruppe an der Formoberfläche des Gusswerkzeugs kann ein Eindringen des flüssigen Kunststoffs in einen Spalt zwischen dem vorbestimmten Bereich der äußeren Oberfläche der Baugruppe und der Formoberfläche des Gusswerkzeugs vermieden werden oder auf einen hinreichend dünnen Film begrenzt sein.

Bei einem Verfahren, wie es hier beschrieben ist, umfasst das Bereitstellen des Schaftrohrs ein Bilden der Verbindungseinrichtung als zungenförmiger Fortsatz des Schaftrohrs, wobei das mechanische Verbinden der Baugruppe mit dem distalen Ende des Schaftrohrs ein mechanisches Verbinden mit einem distalen Ende des zungenförmigen Fortsatzes umfasst.

Das mechanische Verbinden der Baugruppe mit dem distalen Ende des Schaftrohrs erfolgt beispielsweise durch Kleben, Löten oder Schweißen oder formschlüssig, etwa durch einen Crimpvorgang.

Die Ausbildung der Verbindungseinrichtung als zungenförmiger Fortsatz des Schaftrohrs schafft eine besonders einfache und gleichzeitig mechanisch robuste und dauerhafte Verbindungseinrichtung. Beispielsweise wird von einem Endbereich eines Rohrs ein C-förmiger Bereich durch Fräsen oder ein anderes spanabhebendes Verfahren oder durch Laserschneiden oder durch Stanzen oder durch ein anderes Verfahren entfernt, sodass in einem Endabschnitt des ursprünglichen Rohrs nur der zungenförmige Fortsatz verbleibt.

Bei einem Verfahren, wie es hier beschrieben ist, umfasst das Bilden der Verbindungseinrichtung insbesondere ein plastisches Verformen des zungenförmigen Fortsatzes.

Der zungenförmige Fortsatz wird insbesondere von der Mantelfläche des ursprünglichen Rohrs weg und in dessen Lumen hinein verformt. Dies kann eine vollständige Ummantelung der Verbindungseinrichtung durch den Kunststoff innerhalb der nach distal fortgesetzten Kontur des Schaftrohrs ermöglichen.

Ferner kann der zungenförmige Fortsatz plastisch verformt werden, um eine vorbestimmte räumliche Orientierung der mit dem distalen Ende des zungenförmigen Fortsatzes zu verbindenden Baugruppe einzustellen. Ferner kann der zungenförmige Fortsatz verformt werden, um eine formschlüssige Verbindung der Baugruppe mit dem distalen Ende des zungenförmigen Fortsatzes zu ermöglichen oder zu schaffen, beispielsweise in Gestalt einer Crimpverbindung.

Ein Verfahren, wie es hier beschrieben ist, umfasst insbesondere ferner ein mechanisches oder magnetisches Ausüben einer Kraft auf die Baugruppe während des Verfestigens des flüssigen Kunststoffs.

Die Kraft kann durch eine elastische Rückstellkraft der beim Einlegen des Schaftrohrs mit der Baugruppe in das Gusswerkzeug oder beim Schließen des Gusswerkzeugs elastisch verformten Verbindungseinrichtung erzeugt werden. Wenn die Baugruppe selbst oder die Verbindungseinrichtung magnetisch (insbesondere ferromagnetisch) oder magnetisierbar (insbesondere paramagnetisch) ist oder mit einem magnetischen oder magnetisierbaren Körper mechanisch verbunden ist, kann alternativ oder zusätzlich eine Kraft durch einen oder mehrere Magneten ausgeübt werden, die in oder an dem Gusswerkzeug angeordnet sein können.

Alternativ oder zusätzlich kann eine Kraft auf die Baugruppe beispielsweise durch ein Merkmal des Gusswerkzeugs oder durch eine beschleunigte Bewegung, insbesondere als Zentrifugalkraft, mechanisch ausgeübt werden.

Die Kraft kann die Baugruppe während des Verfestigens des flüssigen Kunststoffs und damit dauerhaft in einer vorbestimmten Position halten, in der beispielsweise eine Lichtein- oder - austrittsfläche der Baugruppe an einer Formoberfläche des Gusswerkzeugs anliegt.

Bei einem Verfahren, wie es hier beschrieben ist, schränkt das Verbinden der Baugruppe mit der Verbindungseinrichtung insbesondere einen, zwei, drei, vier, fünf oder sechs Freiheitsgrade der Bewegung der Baugruppe relativ zu dem distalen Ende des Schaftrohrs ein.

Wenn die Verbindungseinrichtung weniger als sechs Freiheitsgrade der Bewegung der Baugruppe relativ zu dem distalen Ende des Schaftrohrs einschränkt, können ein oder mehrere weitere Freiheitsgrade auf andere Weise eingeschränkt werden, beispielsweise durch Ausüben einer Kraft auf die Baugruppe.

Bei einem Verfahren, wie es hier beschrieben ist, umfasst das Einlegen des Bauteils in das Gusswerkzeug insbesondere ein Einlegen einer mit dem Bauteil mechanisch verbundenen Baugruppe, die zumindest entweder einen Bildsensor oder einen anderen Sensor zum Erfassen von einem oder mehreren Messwerten oder eine Leuchtdiode zur Erzeugung von Beleuchtungslicht oder eine andere Lichtquelle oder einen distalen Endbereich eines Arbeits- oder Spülkanals oder eines Lichtwellenleiters oder eine Handhabungseinrichtung aufweist.

Der Bildsensor kann Teil einer Kamera oder einer anderen Einheit aus Objektiv und Bildsensor sein. Die Leuchtdiode kann Teil einer Lichtquelle mit einer oder mehreren Leuchtdioden und einer oder mehreren Linsen oder anderen Einrichtungen zum Formen eines Beleuchtungslichtstrahls sein. Eine Kamera oder eine Leuchtdiode oder eine Lichtquelle wird von dem Kunststoff-Bauteil insbesondere lateral vollständig umschlossen, wobei das Kunststoff-Bauteil eine stoffschlüssige Verbindung mit einem Teil der Oberfläche der Kamera oder der Leuchtdiode oder der Lichtquelle eingeht. Die Baugruppe kann mehrere Bildsensoren und/oder mehrere Leuchtdioden umfassen. Alternativ oder zusätzlich kann die Baugruppe einen oder mehrere andere Sensoren zum Erfassen von Messwerten umfassen, beispielsweise einen Drucksensor zum Erfassen des Drucks innerhalb eines Hohlraums während des Zuspülens eines Fluids oder zu einem anderen Zeitpunkt.

Eine distale Stirnfläche der Kamera, die eine Lichteintrittsfläche umfasst, wird insbesondere nicht von dem Kunststoff-Bauteil bedeckt. Eine distale Stirnfläche einer Leuchtdiode oder einer Lichtquelle, die insbesondere eine Lichtaustrittsfläche umfasst, wird insbesondere nicht von dem Kunststoff-Bauteil bedeckt. Alternativ wird die Lichtaustrittsfläche der Leuchtdiode oder der Lichtquelle von dem Kunststoff-Bauteil bedeckt, nämlich von einer durch das Kunststoff-Bauteil gebildeten Schicht mit einer vorbestimmten Dicke von beispielsweise wenigen Zehnteln eines Millimeters. Diese Schicht kann die Leuchtdiode oder Lichtquelle vor Feuchtigkeit oder anderen Umwelteinflüssen schützen. Eine distale Stirnfläche eines Endbereichs eines Arbeits- oder Spülkanals oder eines Lichtwellenleiters, die insbesondere eine Öffnung zu dem Arbeits- oder Spülkanal oder eine Lichtaustrittsfläche des Lichtwellenleiters umfasst, wird insbesondere nicht von dem Kunststoff-Bauteil bedeckt.

Bei einem Verfahren, wie es hier beschrieben ist, umfasst das Einlegen des Bauteils in das Gusswerkzeug insbesondere ein Einlegen einer elektronischen Baugruppe zur Anordnung an einem proximalen Ende eines Endoskops oder das Einlegen eines Gelenkbereichs.

Das Kunststoff-Bauteil kann die elektronische Baugruppe oder den Gelenkbereich vor einem Fluid schützen und/oder ihm eine vorbestimmte äußere Oberfläche verleihen.

Bei einem Verfahren, wie es hier beschrieben ist, umfasst das Zuführen des flüssigen Kunststoffs insbesondere ein Füllen einer lateral angeordneten Öffnung in dem Schaftrohr.

Die lateral angeordnete Öffnung in dem Schaftrohr kann eine Einkopplung elektromagnetischer Strahlung in das Schaftrohr ermöglichen. Durch das Füllen der lateral angeordneten Öffnung in dem Schaftrohr kann nach dem Verfestigen des flüssigen Kunststoffs gleichzeitig die lateral angeordnete Öffnung geschlossen und eine formschlüssige Verbindung des verfestigten Kunststoffs mit dem Schaftrohr geschaffen werden.

Ein Gusswerkzeug zum Ergänzen eines Bauteils eines medizinischen Instruments durch ein Kunststoff-Bauteil umfasst einen Hohlraum, wobei ein erster Bereich einer Formoberfläche des Hohlraums zum Anliegen an einem Oberflächenbereich des Bauteils vorgesehen ist, wobei ein zweiter Bereich der Formoberfläche des Hohlraums und das Bauteil einen mit Kunststoff zu füllenden Gussraum umschließen, und wobei zumindest entweder ein Werkzeugteil des Gusswerkzeugs oder ein Schieber zum Schließen eines Zuführkanals zum Zuführen von Kunststoff in den Gussraum zumindest teilweise transparent für elektromagnetische Strahlung zum Auslösen oder Bewirken des Bildens des Kunststoff-Bauteils durch Verfestigen eines in den Gussraum gefüllten flüssigen Kunststoffs ausgebildet ist.

Das Gusswerkzeug ist insbesondere für eines der hier beschriebenen Verfahren vorgesehen und ausgebildet. Die hier beschriebenen Verfahren können jedoch auch mit Gusswerkzeugen ausgeführt werden, die andere Merkmale, Eigenschaften und Funktionen aufweisen. Die hier beschriebenen Verfahren können mit dem Gusswerkzeug oder mit anderen Gusswerkzeugen mit abweichenden Merkmalen, Eigenschaften und Verfahren ausgeführt werden.

Das Gusswerkzeug ist insbesondere zur Ergänzung eines metallischen Bauteils eines medizinischen Instruments, beispielsweise eines Endoskops, eines Exoskops oder eines Operationsmikroskops ausgebildet. Das Bauteil kann rohrförmig sein, beispielsweise ein gerades oder gekrümmtes, starres oder teilweise oder vollständig flexibles Schaftrohr. Dabei kann das Gusswerkzeug zum Ergänzen oder Hinzufügen von Kunststoff an einem proximalen oder einem distalen Ende des Schaftrohrs vorgesehen und ausgebildet sein.

Der Hohlraum ist von einem oder mehreren Werkzeugteilen des Gusswerkzeugs weitgehend oder vollständig umschlossen. Der Hohlraum wird bei der vorgesehenen Verwendung während des Gusses teilweise von dem Bauteil eingenommen. Der erste Bereich der Formoberfläche des Hohlraums, der zum Anliegen an einem korrespondierenden Oberflächenbereich des Bauteils vorgesehen ist, kann flächig oder linienförmig sein. Der Gussraum ist insbesondere der nicht von dem Bauteil eingenommene Teil des Hohlraums.

Die zumindest teilweise transparente Ausgestaltung von einem oder mehreren Werkzeugteilen des Gusswerkzeugs oder einem oder mehreren Schiebern zum Schließen von Zuführkanälen ermöglicht eine Einkopplung elektromagnetischer Strahlung, insbesondere ultravioletten Lichts oder Lichts innerhalb des vom gesunden menschlichen Auge als blau wahrgenommenen Spektralbereichs. Das Auslösen oder Bewirken des Verfestigens eines flüssigen Kunststoffs kann ein Ergänzen eines Bauteils bei Raumtemperatur ermöglichen. Schädliche Einflüsse von Temperaturen und Temperaturgradienten können damit vermieden werden.

Ein Werkzeugteil eines Gusswerkzeugs, wie es hier beschrieben ist, weist insbesondere einen Formeinsatz aus einem für die elektromagnetische Strahlung transparenten Material auf.

Der Formeinsatz kann einen Teil des Werkzeugteils bilden und mit diesem fest verbunden oder relativ zu diesem bewegbar sein. Der Formeinsatz bildet zumindest einen Teil des zweiten Bereichs der Formoberfläche des Hohlraums und grenzt somit unmittelbar an den dem Gusswerkzeug zugeführten flüssigen und zu verfestigenden Kunststoff an.

Ein Werkzeugteil eines Gusswerkzeugs, wie es hier beschrieben ist, ist insbesondere aus einem für die elektromagnetische Strahlung transparenten Material gebildet.

Die Ausbildung eines ganzen Werkzeugteils aus einem für die elektromagnetische Strahlung transparenten Material kann eine besonders großflächige Einkopplung der elektromagnetischen Strahlung in den flüssigen und zu verfestigenden Kunststoff ermöglichen.

Bei einem Gusswerkzeug, wie es hier beschrieben ist, weist der Schieber insbesondere einen Bestandteil aus einem für die elektromagnetische Strahlung transparenten Material auf.

Beispielsweise weist der Schieber einen stabförmigen oder zylindrischen Bestandteil aus einem transparenten Material auf, der von einer Metallhülse umgeben ist. Insbesondere bilden sowohl der transparente Bestandteil als auch die Hülse je einen Teil der Oberfläche des Gussraums. Damit grenzt der transparente Bestandteil unmittelbar an den Gussraum an, und elektromagnetische Strahlung kann durch den transparenten Bestandteil in flüssigen und zu verfestigenden Kunststoff in dem Gussraum eingekoppelt werden.

Bei einem Gusswerkzeug, wie es hier beschrieben ist, ist der Schieber insbesondere aus einem für die elektromagnetische Strahlung transparenten Material gebildet.

Sowohl wenn der gesamte Schieber aus einem transparenten Material gebildet ist, als auch wenn der Schieber einen Bestandteil aus einem transparenten Material aufweist, kann das von dem Gussraum abgewandte Ende des Schiebers oder des transparenten Bestandteils des Schiebers durch einen Lichtwellenleiter, ein Lichtleitkabel, eine Linse, einen Spiegel und/oder andere optische Bauelemente mit einer oder mehreren Lichtquellen gekoppelt sein.

Die Ausbildung des gesamten Schiebers oder eines Bestandteils des Schiebers aus einem für die elektromagnetische Strahlung transparenten Material kann eine im Übrigen herkömmliche Ausgestaltung des Gusswerkzeugs ermöglichen, insbesondere eine Ausgestaltung des übrigen Gusswerkzeugs aus einem für die elektromagnetische Strahlung nicht transparenten Material.

Bei einem Gusswerkzeug, wie es hier beschrieben ist, ist die Oberfläche des Gussraums insbesondere aus Polytetrafluorethylen oder Fluorethylenpropylen oder einem amorphen Fluorpolymer oder einem anderen Material, mit dem der Kunststoff keine stoffschlüssige Verbindung eingeht, gebildet.

Polytetrafluorethylen ist auch unter der Bezeichnung Polytetrafluorethen, der Abkürzung PTFE und unter anderem unter dem Markennamen Teflon bekannt. Amorphe Fluorpolymere werden auch unter dem Markennamen Teflon AF vertrieben.

Es kann entweder nur der zweite Bereich der Formoberfläche des Gusswerkzeugs oder auch der erste Bereich der Formoberfläche des Gusswerkzeugs aus einem der genannten Materialien gebildet sein. Dabei kann lediglich eine dünne Beschichtung mit einem der genannten Materialien vorgesehen sein. Alternativ können ein oder mehrere Werkzeugteile des Gusswerkzeugs oder ein oder mehrere Formeinsätze in Werkzeugteilen des Gusswerkzeugs teilweise oder vollständig aus einem der genannten Materialien gebildet sein.

Ein medizinisches Instrument umfasst ein Schaftrohr mit einem distalen Ende, eine Baugruppe an dem distalen Ende des Schaftrohrs, eine Verbindungseinrichtung, die die Baugruppe mit dem Schaftrohr mechanisch verbindet, und eine Kunststoff-Ummantelung, die die Baugruppe zumindest teilweise umgibt, mit der äußeren Oberfläche der Baugruppe stoffschlüssig verbunden ist, die Verbindungseinrichtung umgibt und ebenfalls die Baugruppe mit dem Schaftrohr mechanisch starr verbindet.

Das medizinische Instrument ist insbesondere ein Endoskop oder eine Exoskop oder ein Operationsmikroskop. Das Schaftrohr kann gerade oder gekrümmt, starr oder teilweise oder vollständig flexibel ausgebildet sein. Das Schaftrohr kann das Hauptstrukturelement des Schafts des medizinischen Instruments sein.

Das medizinische Instrument kann insbesondere mittels eines Verfahrens, wie es hier beschrieben ist, und/oder mittels eines Gusswerkzeugs, wie es hier beschrieben ist, hergestellt werden. Ein Verfahren, wie es hier beschrieben ist, und ein Gusswerkzeug, wie es hier beschrieben ist, ist insbesondere zur Herstellung eines medizinischen Instruments, wie es hier beschrieben ist, vorgesehen und ausgebildet.

Die Kunststoff-Ummantelung ist ein Kunststoff-Bauteil, das die Baugruppe insbesondere lateral umgibt. Ein vorbestimmter Oberflächenbereich, insbesondere eine Lichtaustrittsfläche oder eine Lichteintrittsfläche der Baugruppe kann von der Kunststoff-Ummantelung unbedeckt, also nicht bedeckt sein, sondern selbst einen Teil der äußeren Oberfläche des medizinischen Instruments bilden. Die Verbindungseinrichtung ist insbesondere lateral vollständig, also hülsenförmig oder schlauchförmig oder mantelförmig von der Kunststoff-Ummantelung umgeben. Dazu ist die Verbindungseinrichtung insbesondere nicht oder nur teilweise an der äußeren Oberfläche des medizinischen Instruments angeordnet.

Die Verbindungseinrichtung kann schon vor Herstellung der Kunststoff-Ummantelung eine mechanische Verbindung mit der Baugruppe mit dem Schaftrohr schaffen. Dies ermöglicht eine gemeinsame Handhabung der Baugruppe mit dem Schaftrohr und eine vorbestimmte Anordnung der Baugruppe in einem Gusswerkzeug zum Herstellen der Kunststoff-Ummantelung allein durch eine vorbestimmte Anordnung des Schaftrohrs. Das medizinische Instrument weist deshalb beispielsweise keine Ausnehmungen auf, durch die bei einem herkömmlichen Verfahren Stifte, Schieber oder andere mechanische Einrichtungen die Baugruppe während der Herstellung der Kunststoff-Ummantelung in der erwünschten vorbestimmten Position halten.

Bei einem medizinischen Instrument, wie es hier beschrieben ist, ist die Verbindungseinrichtung aus einem zungenförmigen Fortsatz des Schaftrohrs gebildet.

Der zungenförmige Fortsatz ist insbesondere durch Entfernen eines C-förmigen Endbereichs eines Rohrs, sodass lediglich der zungenförmige Fortsatz verbleibt, gebildet.

Die Verbindungseinrichtung kann ferner durch plastisches Verformen, insbesondere Biegen des zungenförmigen Fortsatzes gebildet sein. Das Biegen des zungenförmigen Fortsatzes kann diesen von der nach distal fortgesetzten äußeren Konturen des Schaftrohrs entfernen und eine vollständige Kunststoff-Ummantelung der Verbindungseinrichtung innerhalb der nach distal fortgesetzten Kontur des Schaftrohrs ermöglichen.

Das plastische Verformen des zungenförmigen Fortsatzes kann ferner eine gewünschte Positionierung und Ausrichtung der Baugruppe relativ zu dem distalen Ende des Schaftrohrs ermögliche. Das plastische Verformen des zungenförmigen Fortsatzes kann ferner die mechanischen Verbindung der Baugruppe mit dem zungenförmigen Fortsatz ermöglichen oder schaffen (beispielsweise durch einen Crimpvorgang).

Es können mehrere zungenförmige Fortsätze vorgesehen sein, die jeweils eine Verbindungseinrichtung bilden. Jeder zungenförmige Fortsatz kann eine eigene Baugruppe mit dem distalen Ende des Schaftrohrs mechanisch verbinden. Alternativ können mehrere zungenförmige Fortsätze die selbe Baugruppe mit dem distalen Ende des Schaftrohrs mechanisch verbinden.

Ein medizinisches Instrument, wie es hier beschrieben ist, umfasst insbesondere ferner eine lateral angeordnete Öffnung in dem Schaftrohr, wobei der Kunststoff der Kunststoff-Ummantelung das Schaftrohr zumindest teilweise und die lateral angeordnete Öffnung vollständig ausfüllt.

Die lateral angeordnete Öffnung ist beispielsweise als Querbohrung orthogonal zu einer Längsachse des Schaftrohrs ausgebildet. Es können lediglich eine oder mehrere, insbesondere zwei einander gegenüberliegend angeordnete Öffnungen vorgesehen sein.

Die Öffnung kann bei der Herstellung der KunststoffUmmantelung zum Zuführen von flüssigem Kunststoff und/oder zur Einkopplung elektromagnetischer Strahlung zum Auslösen oder Bewirken einer Verfestigung ursprünglich flüssigen Kunststoffs ermöglichen. Ferner kann der die Öffnung ausfüllende verfestigte Kunststoff eine formschlüssige Verbindung zwischen der Kunststoff-Ummantelung und dem Schaftrohr schaffen.

Bei einem medizinischen Instrument, wie es hier beschrieben ist, umfasst die Baugruppe insbesondere zumindest entweder einen Bildsensor oder eine Leuchtdiode oder eine andere Lichtquelle zur Erzeugung von Beleuchtungslicht oder einen Endbereich eines Lichtwellenleiters oder einen distalen Endbereich eines Arbeits- oder Spülkanals.

Der Endbereich ist insbesondere ein distaler Endbereich des Lichtwellenleiters oder des Arbeits- oder Spülkanals.

Ein medizinisches Instrument, wie es hier beschrieben ist, kann ein Wärmerohr zum Ableiten von Abwärme einer Baugruppe, insbesondere zum Übertragen von Abwärme einer Baugruppe an einem distalen Ende eines Endoskops zu einem proximalen Ende des Endoskops umfassen Das distale Ende des Wärmerohrs ist insbesondere mit der Baugruppe mechanisch verbunden, liegt an dieser an oder ist in unmittelbarer Nähe der Baugruppe mit einem im Verhältnis zu den übrigen Dimensionen der Baugruppe und des Wärmerohrs geringen Abstand angeordnet.

Ein medizinisches Instrument, wie es hier beschrieben ist, umfasst insbesondere ferner ein Wärmerohr, wobei die Verbindungseinrichtung durch das Wärmerohr gebildet oder mit dem Wärmerohr mechanisch und thermisch unmittelbar verbunden ist.

Wärmerohre sind auch im deutschsprachigen Raum unter ihrer englischsprachigen Bezeichnung Heatpipe bekannt. Ein Wärmerohr im Inneren eines Schaftrohrs kann eine Abfuhr von Abwärme der Baugruppe ermöglichen.

Ein medizinisches Instrument, wie es hier beschrieben ist, ist insbesondere ein Endoskop oder ein Laryngoskop mit einem starren oder teilweise oder vollständig flexiblen Schaft.

### Kurzbeschreibung der Figuren

Nachfolgend werden Ausführungsformen anhand der beigefügten Figuren näher erläutert. Es zeigen:
- Figur 1: eine schematische Darstellung eines distalen Endes eines Endoskops;
- Figur 2: eine schematische Darstellung einer Baugruppe für ein proximales Ende eines Endoskops;
- Figur 3: eine schematische Darstellung eines proximalen Endbereichs eines Endoskops;
- Figur 4: eine schematische Darstellung eines Gelenkbereichs eines Endoskops;
- Figur 5: eine schematische Darstellung eines distalen Endes eines weiteren Endoskops;
- Figur 6: eine schematische Darstellung eines Rohrs als Halbzeug für ein Schaftrohr;
- Figur 7: eine schematische Darstellung eines Schaftrohrs mit einer Verbindungseinrichtung;
- Figur 8: eine schematische Darstellung des Schaftrohrs aus Figur 7 mit einer Baugruppe an der Verbindungseinrichtung;
- Figur 9: eine schematische Darstellung des Schaftrohrs und der Baugruppe aus Figur 8 in einem offenen Gusswerkzeug;
- Figur 10: eine weitere schematische Darstellung des Gusswerkzeugs aus Figur 9;
- Figur 11: eine weitere schematische Darstellung des Gusswerkzeugs aus den Figuren 9 und 10;
- Figur 12: eine weitere schematische Darstellung des Gusswerkzeugs aus den Figuren 9 bis 11;
- Figur 13: ein schematisches Flussdiagramm.

### Beschreibung der Ausführungsformen

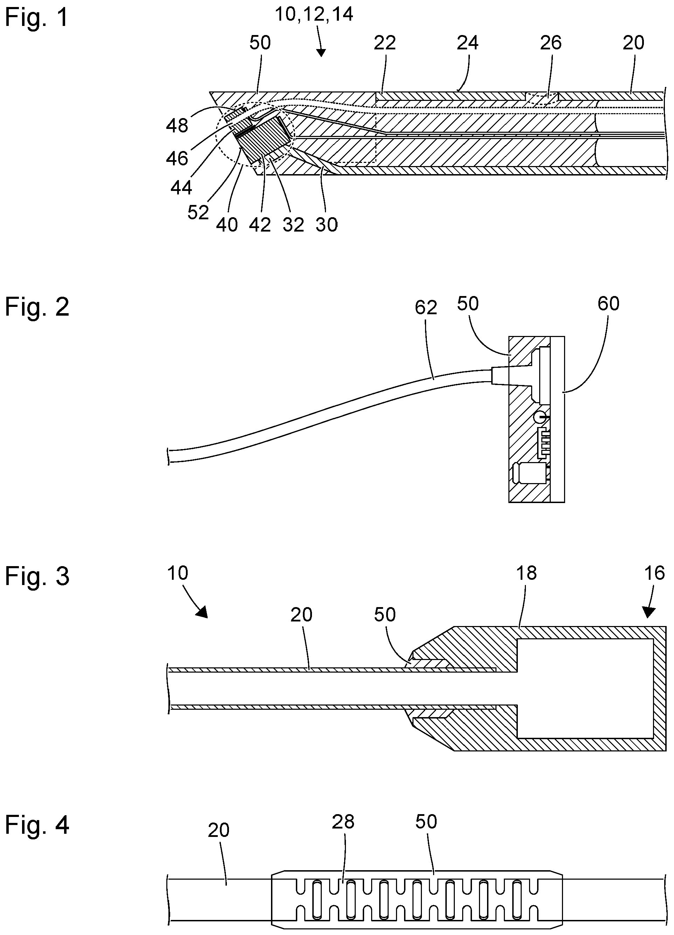

Figur 1 zeigt eine schematische Darstellung eines Schnitts durch einen distalen Endbereich 12 eines Endoskops 10, nämlich des Schafts 14 des Endoskops 10. Der Schaft 14 oder zumindest sein in Figur 1 dargestellter distaler Endbereich 12 ist starr und gerade. Alternativ kann der Schaft 14 abschnittsweise oder vollständig flexibel sein, wobei auch in diesem Fall der distale Endbereich insbesondere starr ausgebildet ist.

Der Schaft 14 umfasst ein Schaftrohr 20 mit einem insbesondere kreisringförmigen Querschnitt. Das Schaftrohr 20 weist ein distales Ende 22 in Form eines kreisringförmigen Randes auf. Bei dem dargestellten Beispiel liegt der das Ende 22 bildende kreisringförmige Rand in einer Ebene orthogonal zu einer Längsachse des Schaftrohrs 20 und orthogonal zu der Zeichenebene der Figur 1 liegt.

Die laterale Oberfläche 24 des Schaftrohrs 20 weist eine zylindermantelförmige Gestalt auf. Die laterale Oberfläche 24 des Schaftrohrs 20 kann wie in Figur 1 angedeutet offen liegen oder abweichend von der Darstellung in Figur 1 von einer oder mehreren weiteren Schichten mit elektrischen, chemischen und/oder mechanischen Funktionen umgeben sein.

Bei dem dargestellten Beispiel weist das Schaftrohr 20 eine laterale Öffnung 26 auf. Die laterale Öffnung 26 ist insbesondere eine Bohrung, deren Achse orthogonal zu der Längsachse des Schaftrohrs 20 ist und diese schneidet.

Ein zungenförmiger Fortsatz 30 setzt das distale Ende 22 des Schaftrohrs 20 an einer Stelle nach distal fort. An einem distalen Ende 32 des zungenförmigen Fortsatzes 30 ist eine Baugruppe 40 befestigt. Bei dem dargestellten Beispiel umfasst die Baugruppe 40 eine Kameraeinheit 42 und eine Lichtquelle 44. Die Kameraeinheit 42 umfasst ein Objektiv zum Erzeugen eines reellen Bilds und einen Bildsensor zum Erfassen des reellen Bilds und zum Erzeugen eines Bildsignals, das das erfasste Bild repräsentiert. Die Lichtquelle 44 umfasst insbesondere eine oder mehrere Leuchtdioden zum Erzeugen von Beleuchtungslicht.

Optional kann die Baugruppe 40 weitere Komponenten umfassen, beispielsweise einen distalen Endbereich 46 eines in Figur 1 durch gestrichelte Linien angedeuteten Arbeits- oder Spülkanals.

Ein lateraler Oberflächenbereich 48 der Baugruppe 40 ist von einer Kunststoff-Ummantelung 50 bedeckt und mit diesem insbesondere stoffschlüssig verbunden. Die Kunststoff- Ummantelung 50 erstreckt sich in proximaler Richtung bis zu dem distalen Ende 22 des Schaftrohrs 20. Ferner ragt die Kunststoff-Ummantelung 50 in das Schaftrohr hinein und füllt dieses bis proximal der lateralen Öffnung 26 aus. Die Kunststoff-Ummantelung füllt dabei auch die laterale Öffnung 26 in dem Schaftrohr 20 aus. Dies schafft eine formschlüssige Verbindung zusätzlich zu der stoffschlüssigen Verbindung zwischen der Kunststoff-Ummantelung 50 einerseits und der distalen Stirnfläche des Schaftrohrs an dessen distalem Ende 22 und der inneren Oberfläche des Schaftrohrs 20 andererseits.

Die Kunststoff-Ummantelung 50 bedeckt nicht eine distale Stirnfläche 52 der Baugruppe 40, die insbesondere eine Lichtaustrittsfläche der Lichtquelle 44, eine Lichteintrittsfläche der Kameraeinheit 42 und gegebenenfalls eine Öffnung des distalen Endbereichs 46 des Arbeits- oder Spülkanals umfasst. Flexible Leiterbahnen und/oder andere elektrische oder optische Leitungen verbinden die Baugruppe 40 durch das Schaftrohr 20 hindurch mit einem in Figur 1 nicht dargestellten proximalen Ende des Endoskops 10.

Zwischen der Kameraeinheit 42 und der Lichtquelle 44 kann ein Licht in Figur 1 als dunkler Strich angedeutete optische Trennschicht vorgesehen sein, die einen unmittelbaren Übertritt von Beleuchtungslicht von der Lichtquelle 44 in die Kameraeinheit 42 unterbindet.

Die Baugruppe 40 ist zweifach mit dem Schaftrohr 20 mechanisch verbunden. Die Kunststoff-Ummantelung 50 ist mit dem Schaftrohr 20 stoffschlüssig und, indem sie auch die laterale Öffnung 26 in dem Schaftrohr 20 ausfüllt, auch formschlüssig verbunden. Ferner ist die Kunststoff-Ummantelung 50 stoffschlüssig und optional auch formschlüssig mit der Baugruppe 40 mechanisch verbunden. Somit verbindet die Kunststoff-Ummantelung 50 die Baugruppe 40 mechanisch starr mit dem Schaftrohr 20. Ferner ist die Baugruppe 40 durch den zungenförmigen Fortsatz mit dem Schaftrohr 20 mechanisch verbunden, und zwar schon vor Bildung der Kunststoff-Ummantelung 50.

Figur 2 zeigt eine schematische Darstellung einer Baugruppe 60, die beispielsweise zur Anordnung in einem Handgriff an einem proximalen Ende eines Mediastinoskops oder eines anderen Endoskops vorgesehen ist. Die Baugruppe 60 weist insbesondere in einer Ebene orthogonal zu der Zeichenebene der Figur 2 eine hufeisenförmige oder halbkreisbogenförmige Gestalt auf.

Die Baugruppe 60 umfasst neben einer Platine mit elektronischen und elektrooptischen Bauelementen eine Kunststoff-Ummantelung 50. Die Kunststoff-Ummantelung 50 bedeckt die Platine und schließt die elektronischen und elektrooptischen Bauelemente ein. Dadurch schützt die Kunststoff-Ummantelung 50 die Platine und die Bauelemente vor mechanischen Einwirkungen, vor physikalischen Einwirkungen (beispielsweise dem Kontakt mit einem spannungsführenden Gegenstand) und vor chemischen Wirkung eines umgebenden Fluids. Ferner umschließt die Kunststoff-Ummantelung 50 ein proximales Ende von Lichtleitfasern 62 verbindet diese stoffschlüssig und/oder formschlüssig mit der Platine. Dadurch schafft die Kunststoff-Ummantelung 50 eine dauerhafte mechanische Verbindung zwischen der Baugruppe 60 und dem proximalen Ende der Lichtleitfasern 62. Dabei verhindert die Kunststoff-Ummantelung insbesondere auch ein Eindringen eines Fluids zwischen die Enden der Lichtleitfasern 62 und einem elektrooptischen Bauteil der Baugruppe 60.

Bei dem dargestellten Beispiel bedeckt die Kunststoff-Ummantelung 50 die Baugruppe 60 nur an einer Seite. Abweichend von der Darstellung in Figur 2 kann die Kunststoff-Ummantelung 50 die Baugruppe 60 an mehreren Seiten bedecken oder vollständig umhüllen.

Die Kunststoff-Ummantelung 50 ist ein Kunststoff-Bauteil, das insbesondere unmittelbar an den übrigen Bestandteilen der Baugruppe 60 durch einen Gussprozess hergestellt und dabei von Anfang an mit diesen stoffschlüssig und/oder formschlüssig verbunden ist.

Figur 3 zeigt eine schematische Darstellung eines proximalen Endbereichs 16 eines Endoskops 10. Der proximale Endbereich 16 wird durch eine Handhabungseinrichtung 18 gebildet. Ein proximales Ende eines Schaftrohrs 20 ist in einer korrespondierenden Ausnehmung in dem distalen Ende der Handhabungseinrichtung 18 angeordnet.

Eine Kunststoff-Ummantelung 50 umschließt den Übergangsbereich zwischen dem Schaftrohr 20 und der Handhabungseinrichtung 18 und füllt einen ringförmigen Spalt zwischen beiden. Die Kunststoff-Ummantelung 50 schafft eine stoffschlüssige mechanische Verbindung zwischen dem proximalen Ende des Schaftrohrs 20 und dem distalen Ende der Handhabungseinrichtung 18 und verhindert ein Eindringen eines Fluids zwischen beiden.

Die Kunststoff-Ummantelung 50 ist ein Kunststoff-Bauteil, das insbesondere unmittelbar in dem ringförmigen Spalt zwischen dem proximalen Ende des Schaftrohrs 20 und dem distalen Ende der Handhabungseinrichtung 18 hergestellt und dabei von Anfang an mit diesen stoffschlüssig und/oder formschlüssig verbunden ist.

Figur 4 zeigt eine schematische Darstellung eines biegeflexiblen Gelenkbereichs eines Schaftrohrs 20 eines medizinischen Instruments. Bei dem dargestellten Beispiel ist der biegeflexible Gelenkbereich 28 des Schaftrohrs 20 durch zahlreiche biegeflexible Stege zwischen zahlreichen Ausnehmungen gebildet. Alternativ und abweichend von der Darstellung in Figur 4 kann der biegeflexible Gelenkbereich 28 des Schafts 14 durch ein oder mehrere Paare von aneinander anliegenden Gleitflächen gebildet sein.

Der biegeflexible Gelenkbereich 28 ist durch eine flexible Kunststoff-Ummantelung 50 ummantelt. Die Kunststoff-Ummantelung verhindert ein Eindringen eines Fluids in den Gelenkbereich 28.

Die Kunststoff-Ummantelung 50 ist ein Kunststoff-Bauteil, das insbesondere unmittelbar an dem biegeflexiblen Gelenkbereich 28 hergestellt und dabei von Anfang an mit diesen stoffschlüssig und/oder formschlüssig verbunden ist.

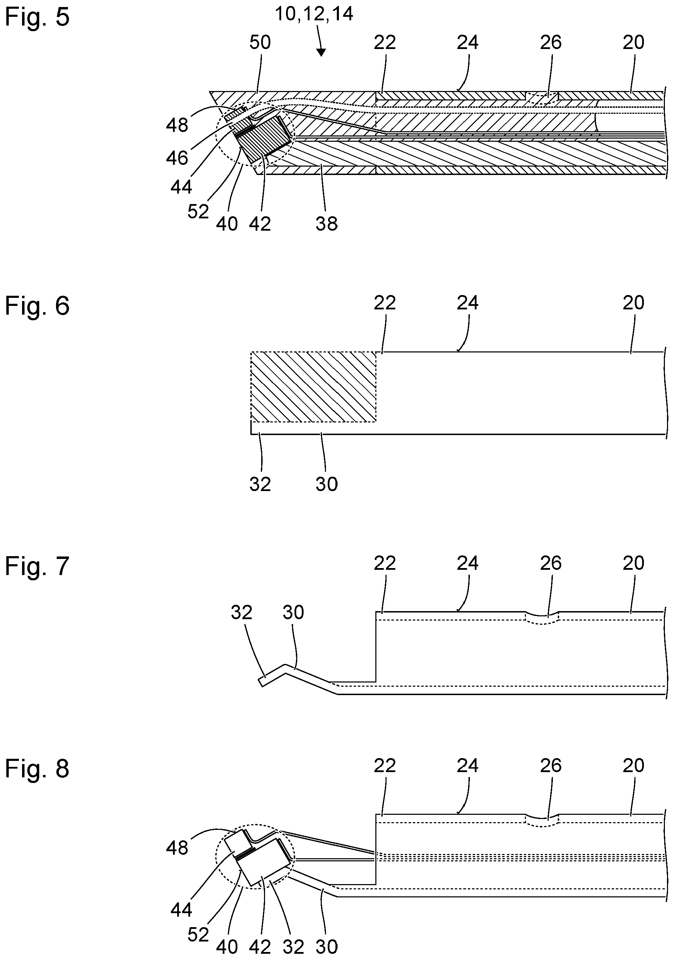

Figur 5 zeigt eine schematische Darstellung eines Schnitts durch einen distalen Endbereich 12 eines Endoskops 10, nämlich des Schafts 14 des Endoskops 10. Die Schnittebene der Figur 5 entspricht der Schnittebene der Figur 1. Das in Figur 5 gezeigte Endoskop 10 ähnelt in einigen Merkmalen, Eigenschaften und Funktionen dem anhand der Figur 1 dargestellten Endoskop. Nachfolgend sind Merkmale, Eigenschaften und Funktionen beschrieben, in denen das in Figur 5 gezeigte Endoskop sich von dem anhand der Figur 1 dargestellten Endoskop unterscheidet.

Das in Figur 5 gezeigte Endoskop 10 unterscheidet sich von dem anhand der Figur 1 dargestellten Endoskop insbesondere dadurch, dass am distalen Ende 22 des Schaftrohrs 20 kein zungenförmiger Fortsatz vorgesehen ist. Stattdessen ist in dem Schaftrohr 20 ein Wärmerohr 38 - auch im deutschsprachigen Raum unter der englischsprachigen Bezeichnung Heatpipe bekannt - angeordnet. Das in Figur 5 nicht sichtbare proximale Ende des Wärmerohrs 38 ist insbesondere an einem proximalen Ende des Endoskops 10 angeordnet. Das distale Ende des Wärmerohrs 38 ist mit der Baugruppe 40 thermisch gekoppelt. Dazu ist das distale Ende des Wärmerohrs 38 mit der Baugruppe 40 insbesondere unmittelbar oder mittelbar mechanisch verbunden. In diesem Fall bildet ein distaler Endbereich des Wärmerohrs 38 gleichzeitig eine Verbindungseinrichtung zur mechanischen Verbindung der Baugruppe 40 mit dem Schaftrohr 20, insbesondere mit dem distalen Ende 22 des Schaftrohrs 20.

Anhand der Figuren 6 bis 13 sind ein Verfahren und eine Gussform zum Herstellen von einer der Kunststoff-Ummantelungen 50 aus den Figuren 1 bis 4 dargestellt. Beispielhaft ist die Herstellung der Kunststoff-Ummantelung 50 an dem anhand der Figur 1 dargestellten distalen Ende 12 eines Endoskops 10 beschrieben.

Figur 6 zeigt eine schematische Darstellung eines Rohrs, aus dem ein Schaftrohr 20 gefertigt wird. Das Rohr weist insbesondere einen kreisringförmigen Querschnitt auf. Die Längsachse, und im Fall eines kreisringförmigen Querschnitts die Symmetrieachse des Rohrs ist parallel zu der Zeichenebene der Figur 6.

Durch eine Schraffur ist ein Bereich angedeutet, der entfernt wird, um das distale Ende 22 des Schaftrohrs 20 und den zungenförmigen Fortsatz 30 freizulegen.

Figur 7 zeigt eine schematische Darstellung des Schaftrohrs 20 in einem späteren Verfahrensstadium. Die Art der Darstellung, insbesondere die Zeichenebene entspricht derjenigen der Figur 6. Anders als in Figur 6 sind die innere Konturen des Schaftrohrs 20 in gestrichelten Linien dargestellt.

Der in Figur 6 schraffiert dargestellte Bereich ist entfernt, sodass das distale Ende 22 des Schaftrohrs 20 und der zungenförmige Fortsatz 30 herausgebildet sind. Der zungenförmige Fortsatz 30 ist plastisch verformt und weist zwei Biegungen auf. Die proximale Biegung des zungenförmigen Fortsatzes schafft einen Abstand zwischen den sich distal anschließenden Bereichen des zungenförmigen Fortsatzes 30 und der durch gestrichelte Linien angedeuteten nach distal fortgesetzten äußeren Mantelfläche des Schaftrohrs 20. Die distale Biegung des zungenförmigen Fortsatzes 30 bewirkt, dass das distale Ende 32 des zungenförmigen Fortsatzes 30 eine vorbestimmte Orientierung relativ zu dem Schaftrohr 20 aufweist.

Figur 8 zeigt eine weitere schematische Darstellung des Schaftrohrs 20. Die Art der Darstellung, insbesondere die Schnittebene entspricht derjenigen der Figur 7. Wie in Figur 7 sind die innere Konturen des Schaftrohrs 20 in gestrichelten Linien dargestellt.

Die Baugruppe 40 mit der Kameraeinheit 42 und der Lichtquelle 44 ist mit dem distalen Ende 32 des zungenförmigen Fortsatzes 30 mechanisch dauerhaft verbunden, beispielsweise durch eine Klebung.

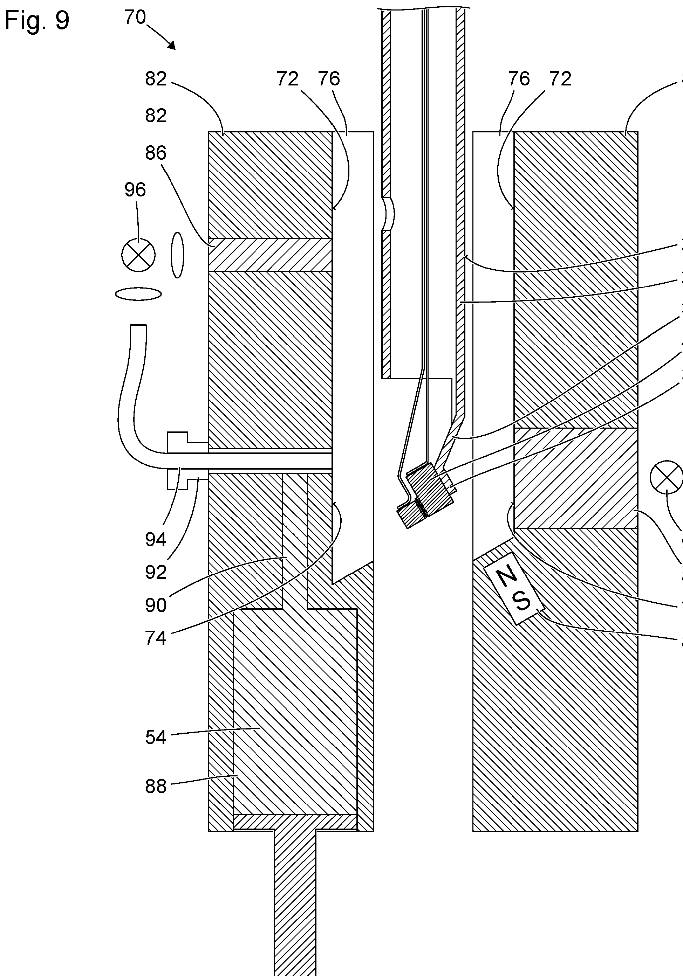

Figur 9 zeigt eine schematische Darstellung der in Figur 8 gezeigten Einheit aus Schaftrohr 20 und Baugruppe 40 am distalen Ende 32 des zungenförmigen Fortsatzes 30 des Schaftrohrs 20. Die Einheit aus Schaftrohr 20 und Baugruppe 40 ist in einem offenen Gusswerkzeug 70 dargestellt. Das Schaftrohr 20, die Baugruppe 40 und das Gusswerkzeug 70 sind in einem Schnitt entlang einer Ebene parallel zu den Zeichenebenen der Figuren 1, 6, 7, 8 dargestellt. Die Schnittebene der Figur 9 enthält die Symmetrieachse des Schaftrohrs 20.

Das Gusswerkzeug 70 weist eine Formoberfläche mit einem ersten Bereich 72 zur flächigen Anlage an der äußeren Oberfläche 24 des Schaftrohrs 20 und einem zweiten Bereich 74 auf. Die Formoberfläche 72, 74 umschließt einen Hohlraum 76, der in dem in Figur 9 dargestellten geöffneten Zustand des Gusswerkzeugs 70 zwei Teilbereiche umfasst.

Das Gusswerkzeug 70 umfasst ein erstes Werkzeugteil 82 und ein zweites Werkzeugteil 84, die jeweils sowohl Anteil an dem ersten Bereich 72 der Formoberfläche als auch an dem zweiten Bereich 74 der Formoberfläche haben. Die Werkzeugteile 82, 84 weisen Formeinsätze 86 auf, die jeweils einen Teil des ersten Bereichs 72 oder des zweiten Bereichs 74 der Formoberfläche des Gusswerkzeugs bilden und bei dem dargestellten Beispiel bis zu äußeren Oberflächenbereichen des Gusswerkzeugs 70 reichen. Die Formeinsätze 86 sind aus für elektromagnetische Strahlung vorbestimmter spektraler Eigenschaften transparenten Materialien gefertigt. Somit kann durch die Formeinsätze 86 elektromagnetische Strahlung in den Hohlraum 76 eingekoppelt werden.

Das Gusswerkzeug 70 weist eine Zuführeinrichtung 88 zum Zuführen von flüssigem Kunststoff 54 zu dem Hohlraum 76 auf. Die Zuführeinrichtung 88 kann aus einem Fluidanschluss bestehen, der mit einer Heiz- und Dosiereinrichtung zum Verflüssigen eines thermoplastischen Kunststoffs und zum Dosieren des verflüssigten Kunststoffs verbunden werden oder verbunden sein kann. Alternativ kann die Zuführeinrichtung eine Heiz- und Dosiereinrichtung zum Verflüssigen und Dosieren eines thermoplastischen Kunststoffs umfassen, die in das Gusswerkzeug 70 integriert sein kann.

Das in Figur 9 dargestellte Gusswerkzeug 70 ist hingegen für einen nicht-thermoplastischen Kunststoff, der bei Raumtemperatur flüssig ist, und dessen Verfestigung durch Einwirkung elektromagnetischer Strahlung in einem vorbestimmten Wellenlängenbereich bewirkt oder ausgelöst wird, vorgesehen. Deshalb umfasst die Zuführeinrichtung 88 keine Heizeinrichtung, sondern lediglich eine Dosiereinrichtung für den flüssigen Kunststoff. Diese Dosiereinrichtung ist durch einen Kolben in einem zylindrischen Hohlraum angedeutet.

Ein Zuführkanal 90 verbindet die Zuführeinrichtung 88 mit dem Hohlraum 76 des Gusswerkzeugs 70. Der Zuführkanal 90 ist bei dem dargestellten Beispiel T-förmig. In dem Zuführkanal 90 ist ein Schieber 92 mit einem optisch transparenten Kern 94 in einer Hülse aus einem mechanisch robusten Material, beispielsweise Stahl, angeordnet. Der Schieber 92 ist in dem Zuführkanal 90 spiel- und reibungsarm geführt und kann verschiedene Positionen einnehmen. In Figur 9 ist der Schieber 92 in einer Position dargestellt, in der sein dem Hohlraum 76 zugewandtes Ende einen Teil der Formoberfläche des Gusswerkzeugs 70, nämlich des zweiten Bereichs 74 der Formoberfläche bildet. Der optisch transparente Kern 94 geht an dem von dem Hohlraum 76 abgewandten Ende in ein Lichtleitkabel über, das mit einer Lichtquelle 96 optisch gekoppelt ist.

In dem zweiten Werkzeugteil 84 des Gusswerkzeugs 70 ist ferner ein Magnet 80 angeordnet. Bei dem dargestellten Beispiel ist der Magnet 80 ein Permanentmagnet, der in das Werkzeugteil 84 eingebettet ist. Alternativ kann der Magnet 80 an einer äußeren Oberfläche des Gusswerkzeugs 70 angeordnet und insbesondere befestigt sein und/oder als Elektromagnet ausgebildet sein.

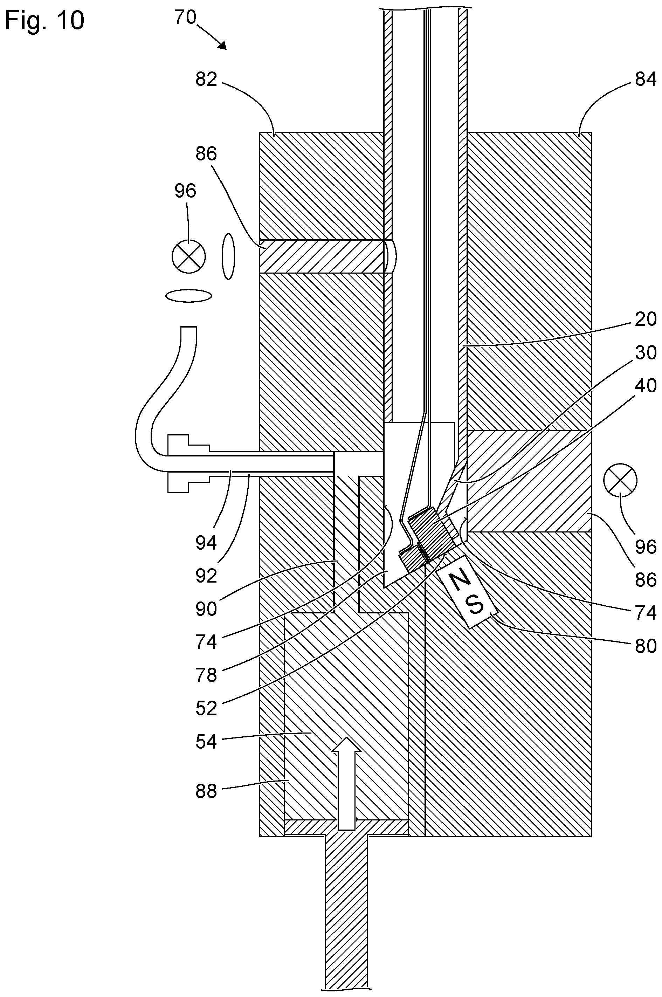

Figur 10 zeigt eine weitere schematische Darstellung der Einheit aus Schaftrohr 20 und Baugruppe 40 und des Gusswerkzeugs 70. Die Art der Darstellung entspricht derjenigen der Figur 9.

In Figur 10 ist das Gusswerkzeug 70 geschlossen dargestellt und die Einheit aus Schaftrohr 20 und Baugruppe 40 ist in dem Hohlraum des Gusswerkzeugs 70 in vorbestimmter Position und Orientierung angeordnet. Dabei liegt die äußere Oberfläche 24 (vgl. Figur 9) des Schaftrohrs 20 flächig an dem ersten Bereich 72 der Formoberfläche des Gusswerkzeugs 70 an. Der zweite Bereich 74 der Formoberfläche des Gusswerkzeugs 70 begrenzt einen Gussraum 78, der in das Lumen des Schaftrohrs 20 übergeht.

In dem Gussraum 78 ist die Baugruppe 40 angeordnet, deren distale Stirnfläche 52 an dem zweiten Bereich 74 der Formoberfläche des Gusswerkzeugs 70 flächig anliegt. Um das flächige Anliegen der distalen Stirnfläche 52 der Baugruppe 40 an dem zweiten Bereich 74 der Formoberfläche des Gusswerkzeugs 70 zu gewährleisten, sind das Schaftrohr 20 so in dem Gusswerkzeug 70 angeordnet und der zungenförmige Fortsatz am distalen Ende 22 des Schaftrohrs 20 so dimensioniert, dass der zungenförmige Fortsatz elastisch verformt ist und seine elastische Rückstellkraft die distale Stirnfläche 52 der Baugruppe 40 gegen den zweiten Bereich 74 der Formoberfläche des Gusswerkzeugs 70 presst. Alternativ oder zusätzlich erzeugt der Magnet 80 eine anziehende Kraft auf das distale Ende 32 des zungenförmigen Fortsatzes 30 am distalen Ende 22 des Schaftrohrs 20 und/oder auf die Baugruppe 40, so dass deren distale Stirnfläche 52 flächig gegen den zweiten Bereich 74 der Formoberfläche des Gusswerkzeugs 70 gepresst ist.

Bei der in Figur 10 gezeigten vorbestimmten Position der Einheit aus Schaftrohr 20 und Baugruppe 40 in dem Gusswerkzeug 70 fluchtet die laterale Öffnung 26 in dem Schaftrohr 20 mit einem der transparenten Formeinsätze 86. Von einer Lichtquelle 96 erzeugte elektromagnetische Strahlung kann deshalb durch diesen transparenten Formeinsatz 86 und durch die laterale Öffnung 26 in das Lumen des Schaftrohrs 20 eingekoppelt werden. Der zweite transparente Formeinsatz 86 ist im Bereich des Gussraums 78 angeordnet, so dass elektromagnetische Strahlung von einer Lichtquelle 96 durch diesen zweiten transparenten Formeinsatz 86 hindurch in den Gussraum 78 eingekoppelt werden kann.

In Figur 10 ist der Schieber 92 in dem Zuführkanal 90 in einer von dem Gussraum 78 entfernten Position dargestellt, in der der Zuführkanal 90 eine offene Verbindung zwischen der Zuführeinrichtung 88 und dem Gussraum 78 bildet. Durch die durch einen Pfeil angedeutete Bewegung des Kolbens der Zuführeinrichtung 88 kann flüssiger Kunststoff aus der Zuführeinrichtung 88 verdrängt und durch den Zuführkanal 90 in den Gussraum 78 gedrückt werden.

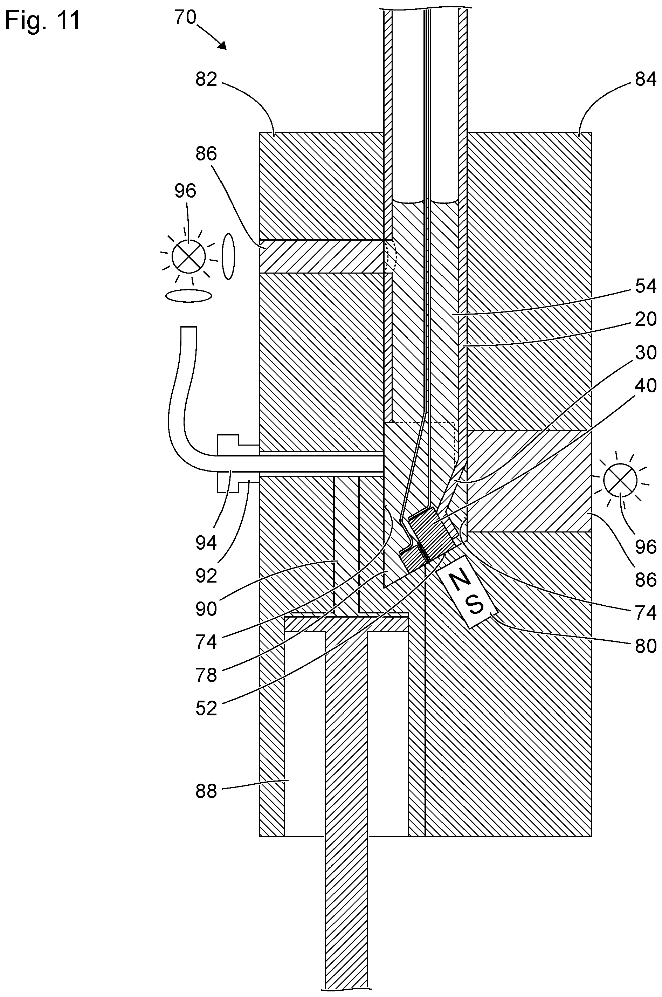

Figur 11 zeigt eine weitere schematische Darstellung der Einheit aus Schaftrohr 20 und Baugruppe 40 in dem Gusswerkzeug 70. Die Art der Darstellung entspricht derjenigen der Figuren 9 und 10.

In Figur 11 ist eine Situation gezeigt, in der der Gussraum 78 mittels der Zuführeinrichtung 88 vollständig und das Lumen des Schaftrohrs 20 so weit mit dem flüssigen Kunststoff gefüllt sind, dass auch die laterale Öffnung 26 in dem Schaftrohr 20 von dem flüssigen Kunststoff 54 ausgefüllt ist. Der Schieber 92 ist wieder ganz in den Zuführkanal 90 eingeführt, so dass sein dem Gussraum 78 zugewandtes Ende einen Teil des zweiten Bereichs 74 der Formoberfläche des Gusswerkzeugs 70 bildet.

In dieser Situation wird den Lichtquellen 96 Leistung zugeführt, um Licht einer vorbestimmten Wellenlänge zu erzeugen, das durch die transparenten Formeinsätze 86 und durch den transparenten Kern 94 des Schiebers 92 in den flüssigen Kunststoff 54 eingekoppelt wird. Das Licht bewirkt eine Verfestigung des flüssigen Kunststoffs 54, beispielsweise durch Polymerisation. Alternativ löst das Licht eine Verfestigung des flüssigen Kunststoffs 54 aus, beispielsweise durch Freisetzung eines Katalysators, der eine Polymerisation bewirkt oder beschleunigt.

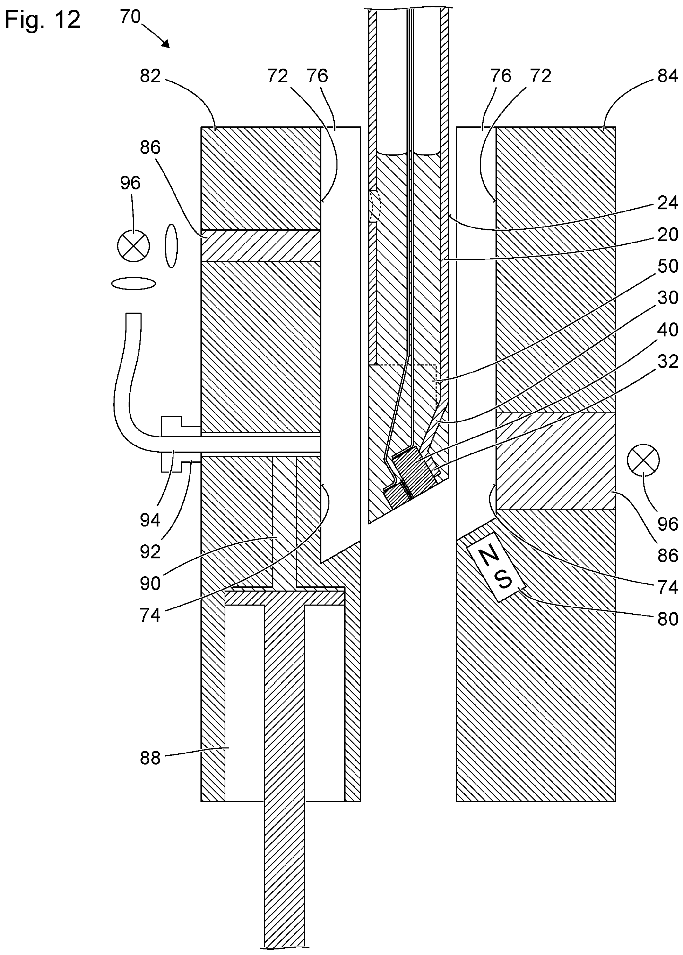

Figur 12 zeigt eine weitere schematische Darstellung der Einheit aus Schaftrohr 20 und Baugruppe 40 und des Gusswerkzeugs 70. Die Art der Darstellung der Figur 12 entspricht derjenigen der Figuren 9 bis 11.

Der flüssige Kunststoff ist durch die Einwirkung elektromagnetischer Strahlung zu der festen Kunststoff-Ummantelung 50 verfestigt, die die Baugruppe 40 und den zungenförmigen Fortsatz 30 umschließt, in das Schaftrohr 20 hineinragt, mit dem Schaftrohr 20 stoffschlüssig und durch Eingriff in die laterale Öffnung 26 in dem Schaftrohr 20 ferner formschlüssig verbunden ist. Das Gusswerkzeug 70 ist geöffnet und das Schaftrohr 20 mit dem fertiggestellten distalen Ende kann entnommen werden.

Für die Herstellung eines Kunststoff-Bauteils oder einer Kunststoff-Ummantelung 50, wie sie anhand der Figuren 2 bis 4 dargestellt sind, weist das Gusswerkzeug Merkmale und Eigenschaften auf, die von den anhand der Figuren 9 bis 12 dargestellten abweichen. Dazu zählt eine entsprechende, nämlich zur herzustellenden äußeren Oberfläche des Kunststoff-Bauteils oder der Kunststoff-Ummantelung 50 korrespondierende geometrische Gestalt des den Gussraum 78 begrenzenden Bereichs 74 der Formoberfläche. Dazu zählt ferner eine entsprechende, nämlich zur lateralen Oberfläche 24 des Schafts 20 (vgl. Figuren 2, 3), der Handhabungseinrichtung 18 (vgl. Figur 3), der Baugruppe 60 und des proximalen Endes der Lichtleitfasern 62 (vgl. Figur 4) korrespondierende geometrische Gestalt des den Gussraum 78 begrenzenden Bereichs 72 der Formoberfläche. Der Hohlraum 76 des Gusswerkzeugs kann dazu wie anhand der Figuren 9 bis 12 dargestellt an einem Ende, durch das das Schaftrohr 20 aus dem Gusswerkzeug heraus reicht, eine Öffnung aufweisen oder an zwei Enden offen sein, so dass beispielsweise im Falle des Ausführungsbeispiels der Figur 4 an beiden Enden heraus ragen kann.

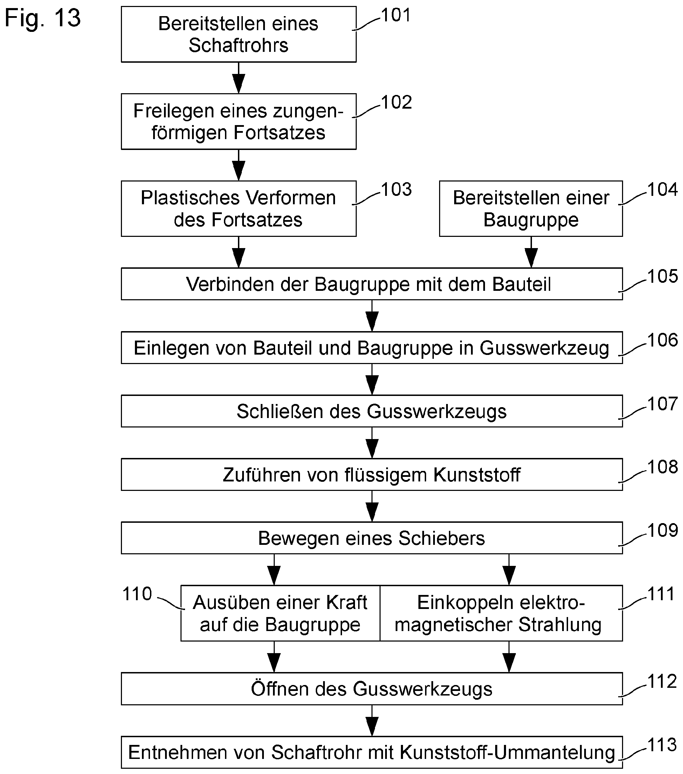

Figur 13 zeigt ein schematisches Flussdiagramm eines Verfahrens zum Ergänzen eines Bauteils eines medizinischen Instruments um ein Kunststoff-Bauteil, insbesondere eine Kunststoff-Ummantelung. Das Verfahren kann mit einem Gusswerkzeug 70 mit den anhand der Figuren 9 bis 12 dargestellten Merkmalen, Eigenschaften und Funktionen oder mit einem Gusswerkzeug mit davon abweichenden Merkmalen, Eigenschaften oder Funktionen ausgeführt werden. Mittels des Verfahrens kann ein medizinisches Instrument mit anhand der Figuren 1, 2, 3, 4, 5 dargestellten Merkmalen, Eigenschaften und Funktionen oder ein medizinisches Instrument mit davon abweichenden Merkmalen, Eigenschaften oder Funktionen hergestellt werden. Beispielhaft werden nachfolgend Bezugszeichen aus den Figuren 1 bis 12 verwendet. Das Verfahren wird beispielhaft anhand eines Schaftrohrs das durch ein Kunststoff-Bauteil zu ergänzen ist, dargestellt.

Bei einem ersten Schritt 101 wird ein zur Bildung eines Schaftrohrs 20 vorgesehenes Rohr bereitgestellt. Bei einem optionalen zweiten Schritt 102 wird ein Teil an einem Ende des Rohrs entfernt, um ein distales Ende 22 des Schaftrohrs 20 und einen zungenförmigen Fortsatz 30 freizulegen bzw. zu bilden. Bei einem optionalen dritten Schritt 103 wird der zungenförmige Fortsatz 30 verformt, insbesondere gebogen.

Alternativ wird ein Schaftrohr 20 mit einem Gelenkbereich versehen. Dies erfolgt beispielsweise durch Bilden von Ausnehmungen, zwischen denen schmale elastische Stege verbleiben. Ein Gelenkbereich kann alternativ ein oder mehrere formschlüssige Gelenke, die jeweils ein Schwenken oder Neigen um eine oder zwei Achsen ermöglichen, umfassen.

Bei einem vierten Schritt 104 wird eine Baugruppe 40 oder eine Handhabungseinrichtung 18 oder eine andere Baugruppe, die mit dem Schaftrohr 20 durch eine Kunststoff-Ummantelung zu verbinden ist, bereitgestellt. Bei einem fünften Schritt wird die Baugruppe 40 oder die Handhabungseinrichtung 18 mit dem Schaftrohr 20 mechanisch verbunden. Dies erfolgt beispielsweise durch eine Klebung oder auch nur durch eine Steckverbindung, die lediglich eine Ausrichtung des Schaftrohrs 20 einerseits und der Baugruppe 40 oder der Handhabungseinrichtung 18 andererseits bewirkt.

Wenn durch das Verfahren ein Gelenkbereich eines Schafts ummantelt werden soll, können auch der vierte Schritt 104 und der fünfte Schritt 105 entfallen.

Bei einem sechsten Schritt 106 wird das Schaftrohr 20 - insbesondere zusammen mit der Baugruppe 40 oder der Handhabungseinrichtung 18 - in ein Gusswerkzeug 70 eingelegt. Bei einem siebten Schritt 107 wird das Gusswerkzeug 70 geschlossen. Danach kann das Schaftrohr 20 an einer Seite oder an zwei von einander abgewandten Seiten aus dem Gusswerkzeug 70 heraus ragen. Im Fall der Verbindung mit einer Handhabungseinrichtung kann auch die Handhabungseinrichtung aus dem Gusswerkzeug 70 heraus ragen.

Bei einem achten Schritt 108 wird einem Gussraum 78 durch einen Zuführkanal 90 flüssiger Kunststoff zugeführt. Bei einem neunten Schritt 109 wird ein Schieber 92 in dem Zuführkanal 90 bewegt, um den Zuführkanal zu schließen.

Bei einem optionalen zehnten Schritt 110 wird eine Kraft auf die Baugruppe 40 ausgeübt, um diese in eine vorbestimmte Position zu bewegen, beispielsweise flächig an einem Bereich 74 der Formoberfläche des Gusswerkzeugs 70 anliegend. Die Kraft kann beispielsweise eine elastische Rückstellkraft eines zungenförmigen Fortsatzes als mechanischer Verbindungseinrichtung aufgrund einer elastischen Verformung beim Einlegen 106 und/oder Schließen 107. Alternativ kann die Kraft beispielsweise durch einen Magneten erzeugt werden.

Abweichend von der Darstellung in Figur 13 kann die Kraft bereits beginnend vor dem Zuführen des flüssigen Kunststoffs ausgeübt werden, um ein Eindringen des flüssigen Kunststoffs in einen Spalt zwischen Baugruppe und Formoberfläche zu verhindern.

Bei einem während des zehnten Schritts 110 ausgeführten elften Schritt 111 wird elektromagnetische Strahlung in den flüssigen Kunststoff 54 eingekoppelt, um eine Verfestigung des flüssigen Kunststoffs 54 zu einer festen Kunststoff-Ummantelung 50 zu bewirken oder auszulösen.

Die im zehnten Schritt ausgeübte Kraft wird insbesondere mindestens bis zur vollständigen Verfestigung des ursprünglich flüssigen Kunststoffs ausgeübt.

Bei einem zwölften Schritt 112 wird das Gusswerkzeug 70 geöffnet. Bei einem dreizehnten Schritt 113 wird das Schaftrohr 20 mit der Kunststoff-Ummantelung 50 entnommen.

### Bezugszeichen

- **10**: **medizinisches Instrument**
- 12: distaler Endbereich des medizinischen Instruments 10
- 14: Schaft des medizinischen Instruments 10
- 16: proximaler Endbereich des medizinischen Instruments 10
- 18: Handhabungseinrichtung am dem proximalen Endbereich 16 des medizinischen Instruments 10
- **20**: **Schaftrohr**
- 22: distales Ende des Schaftrohrs 20
- 24: laterale Oberfläche des Schaftrohrs 20
- 26: laterale Öffnung in dem Schaftrohr 20
- 28: biegeflexibler Gelenkbereich des Schafts 14
- **30**: **zungenförmiger Fortsatz** am distalen Ende 22 des Schaftrohrs 20
- 32: distales Ende des zungenförmigen Fortsatzes 30
- 38: Wärmerohr
- **40**: **Baugruppe** an dem distalen Ende 12 des medizinischen Instruments 10
- 42: Kameraeinheit
- 44: Lichtquelle, insbesondere Leuchtdiode, zum Erzeugen von Beleuchtungslicht
- 46: distaler Endbereich eines Arbeits- oder Spülkanals
- 48: lateraler Oberflächenbereich der Baugruppe 40
- 50: Kunststoff-Ummantelung der Baugruppe 40 oder 60 oder des biegeflexiblen Gelenkbereichs 28
- 52: distale Stirnfläche der Baugruppe 40
- 54: flüssiger Kunststoff
- 60: Baugruppe an dem proximalen Ende 16 des medizinischen Instruments 10
- 62: Lichtleitfasern, mit der Baugruppe 60 optisch und mechanisch verbunden
- 70: Gusswerkzeug
- 72: erster Bereich der Formoberfläche des Gusswerkzeugs 70
- 74: zweiter Bereich der Formoberfläche des Gusswerkzeugs 70
- 76: Hohlraum des Gusswerkzeugs 70, von dem ersten Bereich 72 und dem zweiten Bereich 74 der Formoberfläche begrenzt
- 78: Gussraum, von dem zweiten Bereich 74 der Formoberfläche und dem Bauteil 20 begrenzt
- 80: Magnet
- 82: erstes Werkzeugteil des Gusswerkzeugs 70
- 84: zweites Werkzeugteil der Gusswerkzeugs 70
- 86: transparenter Formeinsatz des zweiten Werkzeugteils 84
- 88: Zuführeinrichtung für flüssigen Kunststoff
- 90: Zuführkanal zur Zufuhr von flüssigem Kunststoff in den Gussraum 78
- 92: Schieber in dem Zuführkanal 90
- 94: optisch transparenter Kern des Schiebers 92
- 96: Lichtquelle
- 101: erster Schritt (Bereitstellen eines Schaftrohrs)
- 102: zweiter Schritt (Freilegen eines zungenförmigen Fortsatzes und eines distalen Endes des Schaftrohrs)
- 103: dritter Schritt (plastisches Verformen des zungenförmigen Fortsatzes)
- 104: vierter Schritt (Bereitstellen einer Baugruppe)
- 105: fünfter Schritt (mechanisches Verbinden der Baugruppe mit dem zungenförmigen Fortsatz)
- 106: sechster Schritt (Einlegen des Bauteils mit der Baugruppe in ein Gusswerkzeug)
- 107: siebter Schritt (Schließen des Gusswerkzeugs)
- 108: achter Schritt (Zuführen von flüssigem Kunststoff)
- 109: neunter Schritt (Bewegen eines Schiebers, der einen Zuführkanal zum Zuführen von flüssigem Kunststoff verschließt)
- 110: zehnter Schritt (Ausüben einer Kraft auf die Baugruppe)
- 111: elfter Schritt (Einkoppeln einer elektromagnetischen Strahlung in den flüssigen Kunststoff, um ein Verfestigen auszulösen)
- 112: zwölfter Schritt (Öffnen des Gusswerkzeugs)
- 113: dreizehnter Schritt (Entnehmen des Schaftrohrs mit Kunststoff-Ummantelung aus verfestigtem Kunststoff)

## Patentansprüche

1. **Verfahren zum Ergänzen** eines Schaftrohrs und einer Baugruppe eines medizinischen Instruments (10) durch ein Kunststoff-Bauteil, mit folgenden Schritten:
Bereitstellen (101, 102) eines **Schaftrohrs** (20) mit einem distalen Ende (22);
Bilden eines **zungenförmigen Fortsatzes** (30) des Schaftrohrs (20) an dem distalen Ende (22) des Schaftrohrs (20) als Verbindungseinrichtung;
wobei der zungenförmige Fortsatz durch Entfernen eines C-förmigen Endbereichs des Schaftrohrs gebildet wird, sodass der zungenförmige Fortsatz das distale Ende des Schaftrohrs an einer Stelle nach distal fortsetzt,
mechanisches Verbinden (105) einer **Baugruppe** (40) mit einem distalen Ende (32) des zungenförmigen Fortsatzes (30) des distalen Endes (22) des Schaftrohrs (20);
Einlegen (106) des Schaftrohrs (20) mit der Baugruppe (40) in ein Gusswerkzeug (70) und **Schließen** (107) des Gusswerkzeugs (70), wobei ein vorbestimmter Bereich (52) der äußeren Oberfläche der Baugruppe (40) an einer Formoberfläche (72, 74) des Gusswerkzeugs (70) anliegt;
**Zuführen** (108) eines flüssigen Kunststoffs in das Gusswerkzeug (70), wobei der flüssige Kunststoff das distale Ende (22) des Schaftrohrs (20) und die äußere Oberfläche (48) der Baugruppe (40) benetzt;
Verfestigen (111) des flüssigen Kunststoffs,
wobei der verfestigte Kunststoff das Kunststoff-Bauteil bildet und eine weitere mechanische Verbindung der Baugruppe (40) mit dem Ende (22) des Schaftrohrs (20) schafft.

2. Verfahren gemäß dem vorangehenden Anspruch, bei dem
zumindest entweder das Einlegen (106) des Schaftrohrs (20) mit der Baugruppe (40) oder das Schließen (107) des Gusswerkzeugs (70) ein **elastisches Verformen** der Verbindungseinrichtung (30, 38) umfasst, so dass eine **elastische Rückstellkraft** der Verbindungseinrichtung (30, 38) den vorbestimmten Bereich (52) der äußeren Oberfläche der Baugruppe (40) gegen die Formoberfläche (74) des Gusswerkzeugs (70) presst.

3. Verfahren gemäß einem der vorangehenden Ansprüche, bei dem
das Bilden der Verbindungseinrichtung ein plastisches Verformen (103) des zungenförmigen Fortsatzes (30) umfasst.

4. Verfahren gemäß einem der vorangehenden Ansprüche, ferner mit folgendem Schritt:
mechanisches oder magnetisches Ausüben (110) einer **Kraft** auf die Baugruppe (40) während des Verfestigens (111) des flüssigen Kunststoffs.

5. Verfahren gemäß einem der vorangehenden Ansprüche, bei dem
das Zuführen (108) des flüssigen Kunststoffs ein Füllen einer **lateral angeordneten Öffnung** (26) in dem Schaftrohr (20) umfasst.

6. **Medizinisches Instrument** (10), mit:
einem **Schaftrohr** (20) mit einem distalen Ende (22);
einer **Baugruppe** (40) an dem distalen Ende (22) des Schaftrohrs (20);
einer **Verbindungseinrichtung** (30; 38), die die Baugruppe (40) mechanisch mit dem Schaftrohr (20) verbindet;
einer **Kunststoff-Ummantelung** (50), die die Baugruppe (40) zumindest teilweise umgibt, mit der äußeren Oberfläche (48) der Baugruppe (40) stoffschlüssig verbunden ist, die Verbindungseinrichtung (30) umgibt und ebenfalls die Baugruppe (40) mit dem Schaftrohr (20) mechanisch starr verbindet,
**dadurch gekennzeichnet, dass** die Verbindungseinrichtung aus einem **zungenförmigen Fortsatz** (30) des Schaftrohrs (20) gebildet ist und die Baugruppe (40) mit dem distalen Ende des zungenförmigen Fortsatzes (30) mechanisch verbunden ist, wobei der zungenförmige Fortsatz durch Entfernen eines C-förmigen Endbereichs des Schaftsrohrs gebildet wird, sodass der zungenförmige Fortsatz das distale Ende des Schaftrohrs an einer Stelle nach distal fortsetzt.

7. Medizinisches Instrument (10) gemäß dem vorangehenden Anspruch, ferner mit:
einer lateral angeordneten Öffnung (26) in dem Schaftrohr (20),
wobei der Kunststoff der Kunststoff-Ummantelung (50) das Schaftrohr (20) zumindest teilweise und die lateral angeordnete Öffnung (26) vollständig **ausfüllt.**

8. Medizinisches Instrument (10) gemäß einem der Ansprüche 6 und 7, bei dem die Baugruppe (40) zumindest entweder einen **Bildsensor** (42) oder eine **Leuchtdiode** (44) oder eine andere **Lichtquelle** zur Erzeugung von Beleuchtungslicht oder einen distalen Endbereich eines **Lichtwellenleiters** oder einen Endbereich (46) eines **Arbeits-** oder **Spülkanals** umfasst.

9. Medizinisches Instrument (10) gemäß einem der Ansprüche 6 bis 8, ferner mit:
einem **Wärmerohr** (38),
wobei die Verbindungseinrichtung mit dem Wärmerohr (38) mechanisch und thermisch unmittelbar verbunden ist.

10. Medizinisches Instrument (10) gemäß einem der Ansprüche 6 bis 9, wobei
das medizinische Instrument (10) ein Endoskop oder ein Laryngoskop mit einem starren oder teilweise oder vollständig flexiblen Schaft (14) ist.

## Claims

1. A method for supplementing a shaft tube and an assembly of a medical instrument (10) with a plastic component, having the following steps:
providing (101, 102) a shaft tube (20) with a distal end (22);
forming a tongue-shaped extension (30) of the shaft tube (20) at the distal end (22) of the shaft tube (20) as a connecting device;
wherein the tongue-shaped extension is formed by removing a C-shaped end region of the shaft tube such that the tongue-shaped extension continues the distal end of the shaft tube distally at one point,
mechanically connecting (105) an assembly (40) to a distal end (32) of the tongue-shaped extension (30) of the distal end (22) of the shaft tube (20);
inserting (106) the shaft tube (20) with the assembly (40) into a casting mould (70) and closing (107) the casting mould (70), wherein a predetermined region (52) of the outer surface of the assembly (40) rests against a mould surface (72, 74) of the casting mould (70);
supplying (108) a liquid plastic into the casting mould (70), wherein the liquid plastic wets the distal end (22) of the shaft tube (20) and the outer surface (48) of the assembly (40);
solidifying (111) the liquid plastic,
wherein the solidified plastic forms the plastic component and creates a further mechanical connection of the assembly (40) to the end (22) of the shaft tube (20).

2. The method according to the preceding claim, in which at least either inserting (106) the shaft tube (20) with the assembly (40) or closing (107) the casting mould (70) comprises an elastic deformation of the connecting device (30, 38), such that an elastic restoring force of the connecting device (30, 38) presses the predetermined region (52) of the outer surface of the assembly (40) against the mould surface (74) of the casting mould (70).

3. The method according to one of the preceding claims, in which forming the connecting device comprises a plastic deformation (103) of the tongue-shaped extension (30).

4. The method according to one of the preceding claims, further comprising the following step: mechanically or magnetically applying (110) a force on the assembly (40) during solidification (111) of the liquid plastic.

5. The method according to one of the preceding claims, in which supplying (108) the liquid plastic comprises filling a laterally arranged opening (26) in the shaft tube (20).

6. A medical instrument (10), comprising:
a shaft tube (20) with a distal end (22);
an assembly (40) at the distal end (22) of the shaft tube (20);
a connecting device (30; 38) which mechanically connects the assembly (40) to the shaft tube (20);
a plastic covering (50), which at least partially surrounds the assembly (40), is connected in a materially-bonded manner to the outer surface (48) of the assembly (40), surrounds the connecting device (30) and also connects the assembly (40) mechanically and rigidly to the shaft tube (20),
**characterised in that** the connecting device is formed from a tongue-shaped extension (30) of the shaft tube (20) and the assembly (40) is mechanically connected to the distal end of the tongue-shaped extension (30), wherein the tongue-shaped extension is formed by removing a C-shaped end region of the shaft tube, such that the tongue-shaped extension continues the distal end of the shaft tube distally at one point.

7. The medical instrument (10) according to the preceding claim, further comprising: a laterally arranged opening (26) in the shaft tube (20),
wherein the plastic of the plastic covering (50) at least partially fills the shaft tube (20) and completely fills the laterally arranged opening (26).

8. The medical instrument (10) according to one of claims 6 and 7, in which the assembly (40) comprises at least either an image sensor (42) or a light-emitting diode (44) or another light source for generating illumination light or a distal end region of an optical fibre or an end region (46) of a working or irrigation channel.

9. The medical instrument (10) according to one of claims 6 to 8, further comprising: a heat pipe (38),
wherein the connecting device is directly connected mechanically and thermally to the heat pipe (38).

10. The medical instrument (10) according to one of claims 6 to 9, wherein the medical instrument (10) is an endoscope or a laryngoscope with a rigid or partially or fully flexible shaft (14).

## Revendications

1. Procédé permettant de compléter un tube d'arbre et un ensemble d'un instrument médical (10) par un composant en plastique, comportant les étapes suivantes:
mise à disposition (101, 102) d'un tube d'arbre (20) comportant une extrémité distale (22);
formation d'un prolongement en forme de languette (30) du tube d'arbre (20) au niveau de l'extrémité distale (22) du tube d'arbre (20) en tant que dispositif de liaison;
dans lequel le prolongement en forme de languette est formé en retirant une zone d'extrémité en forme de C du tube d'arbre, de sorte que le prolongement en forme de languette prolonge de manière distale l'extrémité distale du tube d'arbre au niveau d'un emplacement,
liaison mécanique (105) d'un ensemble (40) à une extrémité distale (32) du prolongement en forme de languette (30) de l'extrémité distale (22) du tube d'arbre (20);
insertion (106) du tube d'arbre (20) avec l'ensemble (40) dans un outil de coulée (70) et fermeture (107) de l'outil de coulée (70), dans lequel une zone prédéterminée (52) de la surface externe de l'ensemble (40) repose sur une surface de moulage (72, 74) de l'outil de coulée (70);
alimentation (108) d'un plastique liquide dans l'outil de coulée (70), dans lequel le plastique liquide mouille l'extrémité distale (22) du tube d'arbre (20) et la surface externe (48) de l'ensemble (40);
solidification (111) du plastique liquide,
dans lequel le plastique solidifié forme le composant en plastique et crée une autre liaison mécanique de l'ensemble (40) avec l'extrémité (22) du tube d'arbre (20).

2. Procédé selon la revendication précédente, dans lequel au moins soit l'insertion (106) du tube d'arbre (20) avec l'ensemble (40), soit la fermeture (107) de l'outil de coulée (70) comprend une déformation élastique du dispositif de liaison (30, 38), de sorte qu'une force de rappel élastique du dispositif de liaison (30, 38) presse la zone prédéterminée (52) de la surface externe de l'ensemble (40) contre la surface de moulage (74) de l'outil de coulée (70).

3. Procédé selon l'une des revendications précédentes, dans lequel la formation du dispositif de liaison comprend une déformation plastique (103) du prolongement en forme de languette (30).

4. Procédé selon l'une des revendications précédentes, comportant en outre l'étape suivante : exercice mécanique ou magnétique (110) d'une force sur l'ensemble (40) pendant la solidification (111) du plastique liquide.

5. Procédé selon l'une des revendications précédentes, dans lequel l'alimentation (108) du plastique liquide comprend un remplissage d'une ouverture (26) agencée latéralement dans le tube d'arbre (20).

6. Instrument médical (10), comportant :
un tube d'arbre (20) comportant une extrémité distale (22);
un ensemble (40) au niveau de l'extrémité distale (22) du tube d'arbre (20);
un dispositif de liaison (30; 38) qui relie mécaniquement l'ensemble (40) au tube d'arbre (20);
une gaine en plastique (50) qui entoure au moins partiellement l'ensemble (40), est reliée par complémentarité de matière à la surface externe (48) de l'ensemble (40), entoure l'appareil de liaison (30) et relie également mécaniquement rigidement l'ensemble (40) au tube d'arbre (20),
**caractérisé en ce que** l'appareil de liaison est formé d'un prolongement en forme de languette (30) du tube d'arbre (20) et l'ensemble (40) est relié mécaniquement à l'extrémité distale du prolongement en forme de languette (30), dans lequel le prolongement en forme de languette est formé par retrait d'une région d'extrémité en forme de C du tube d'arbre, de sorte que le prolongement en forme de languette prolonge de manière distale l'extrémité distale du tube d'arbre en un emplacement.

7. Instrument médical (10) selon la revendication précédente, comportant en outre : une ouverture (26) agencée latéralement dans le tube d'arbre (20),
dans lequel le plastique de la gaine en plastique (50) remplit au moins partiellement le tube d'arbre (20) et remplit entièrement l'ouverture (26) agencée latéralement.

8. Instrument médical (10) selon l'une des revendications 6 et 7, dans lequel
l'ensemble (40) comprend au moins soit un capteur d'image (42), soit une diode électroluminescente (44), soit une autre source de lumière pour générer de la lumière d'éclairage, soit une zone d'extrémité distale d'une fibre optique, soit une zone d'extrémité (46) d'un canal de travail ou de rinçage.

9. Instrument médical (10) selon l'une des revendications 6 à 8, comportant en outre : un caloduc (38),
dans lequel l'appareil de liaison est relié mécaniquement et thermiquement directement au caloduc (38).

10. Instrument médical (10) selon l'une des revendications 6 à 9, dans lequel l'instrument médical (10) est un endoscope ou un laryngoscope comportant un arbre (14) rigide ou partiellement ou totalement flexible.
